# EUROPEAN PATENT SPECIFICATION

(11) **EP 1 645 302 B1**
(45) Date of publication and mention of the grant of the patent: **14.02.2007**
(21) Application number: 04256241.3
(22) Date of filing: 08.10.2004
(51) Int. Cl.: A61M 5/32

(54) **Disposable syringe with a retractable needle**
Einwegspritze mit zurückziehbarer Nadel
Seringue jetable à aiguille rétractable

(43) Date of publication of application: 12.04.2006
(73) Proprietor: Shue, Ming-Jeng, Hsi Dist, Taichung City (TW); Huang, Deborah, Taichung City (TW); Shue, Philip, Hsi District Taichung City (TW)
(72) Inventor: Shue, Ming-Jeng, Hsi Dist, Taichung City (TW); Huang, Deborah, Taichung City (TW); Shue, Philip, Hsi District Taichung City (TW)
(74) Representative: Godwin, Edgar James

(56) References cited:
- EP-A- 0 979 660
- US-A- 5 318 536
- US-A- 5 938 641
- US-A1- 2004 153 035

## Description

This invention relates to a disposable syringe, more particularly to a disposable syringe with a friction diminishing means to facilitate retraction of a needle into a barrel for safe disposal.

Conventional syringes, especially those with a sharp needle, have to be disposed safely after injection. Although a tip protector is used to shield the needle, the user is exposed to the risk of being stuck by the needle when sleeving the tip protector back on the syringe. There are many syringes with a retractable needle that is retracted into a barrel by pulling a plunger backward. However, it is desirable to improve the steady retraction movement of the needle and to reduce defective products during manufacture.

A disposable syringe of the prior art is shown in document US 2004/0153035.

The object of the present invention is to provide a disposable syringe which permits steady and successful retraction of a needle, and which can reduce failure in production.

According to this invention, the disposable syringe includes a barrel, a tubular needle seat and a plunger. The barrel has an axially extending surrounding barrel wall which defines a passage with front and rear open ends. An inner wall surface of the surrounding barrel wall includes front and rear surface segments and an intermediate surface segment disposed therebetween. The intermediate surface segment has a retaining region axially distal from the front surface segment, and first and second friction diminishing regions respectively interposed between the retaining region and the front surface segment, and between the retaining region and the rear surface segment.

The tubular needle seat is insertable into the passage from the rear open end, and includes a front engaging portion in fluid-tight engagement with the front surface segment to fix a needle cannula, a retaining portion which is disposed rearwardly of the front engaging portion, and which is retained at the retaining region by virtue of a first frictional force when the needle seat is in a position of use, a rear engaging portion which extends rearwardly from the retaining portion to terminate at a rearwardly facing wall, and a surrounding sealing flange which extends from the rearwardly facing wall radially and outwardly to be in fluid-tight engagement with the rear surface segment, and which extends forwardly to terminate at a surrounding flange surface that is movable towards the second friction diminishing region. The rear engaging portion has an inner tubular wall surface defining a cavity which extends from the rearwardly facing wall to terminate at a ceiling wall. The ceiling wall has an axial hole which establishes a fluid communication between the needle cannula and the cavity. A radially yieldable catch is disposed on the inner tubular wall surface distal from the ceiling wall, and is yieldable radially and outwardly in response to a kinetic frictional force.

The plunger is movable in the passage, and has a front end wall, an engaging head opposite to the front end wall, and a neck interposed therebetween and of a dimension such that a rearwardly facing shoulder wall is formed between the engaging head and the neck. The kinetic frictional force is generated as a result of axial movement of the engaging head relative to the radially yieldable catch towards the ceiling wall. A deformable sealing member is sleeved on the engaging head and the neck, is in frictional engagement with the engaging head with a second frictional force, and is sealingly slidable relative to the rear surface segment.

In the position of use, the deformable sealing member is moved forward by virtue of forward movement of the plunger to abut against the rearwardly facing wall, while the engaging head is extended into the cavity.

When the plunger is to be placed in a disposal position, the engaging head is kept moving towards the ceiling wall by a pushing force which is applied to the plunger, and which, when the deformable sealing member is blocked by the rearwardly facing wall from moving with the engaging head, overcomes the second frictional force, thereby exposing the neck so as to permit the rearwardly facing shoulder wall to be forced to slip over the radially yieldable catch and to be prevented from moving rearwardly by the radially yieldable catch.

After the rearwardly facing shoulder wall has slipped over the radiallyyieldable catch, continuedmovement of the engaging head towards the ceiling wall, against the first frictional force, forces the retaining portion and the surrounding sealing flange to move past the first and second friction diminishing regions, respectively, so as to facilitate a subsequent pulling action of the plunger whereby the needle seat is brought towards the rear open end by virtue of the retaining engagement of the rearwardly facing shoulderwall and the radially yieldable catch, thereby retracting the needle cannula into the passage.

Other features and advantages of the present invention will become apparent in the following detailed description of the preferred embodiments of the invention, with reference to the accompanying drawings, in which:
Fig. 1 is an exploded sectional view of the first preferred embodiment of a disposable syringe according to this invention;
Fig. 2 is a sectional view of the first preferred embodiment in a state of use;
Fig. 3 is a fragmentary sectional view of the first preferred embodiment, showing a needle seat retained in a barrel;
Fig. 4 is a sectional view of the first preferred embodiment in an injection completed state;
Fig. 5 is a fragmentary sectional view of the first preferred embodiment showing an engaging head of a plunger retained in a cavity of the needle seat;
Fig. 6 is a sectional view of the first preferred embodiment in a disposal state;
Fig. 7 is a fragmentary sectional viewof the second preferred embodiment of a disposable syringe according to this invention;
Fig. 8 is a fragmentary sectional view of the third preferred embodiment of a disposable syringe according to this invention;
Fig. 9 is a fragmentary sectional viewof the fourthpreferred embodiment of a disposable syringe according to this invention, showing a needle seat retained in a barrel;
Fig. 10 is a fragmentary sectional viewof the fifthpreferred embodiment of a disposable syringe according to this invention, showing a needle seat retained in a barrel;
Fig. 11 is a fragmentary exploded sectional view of the sixth preferred embodiment of a disposable syringe according to this invention, showing three modified forms of needle seats for selective use with a barrel;
Fig. 12 is an exploded sectional view of the sixth preferred embodiment in a disposal state;
Fig. 13 is a fragmentary exploded sectional view of the seventh preferred embodiment of a disposable syringe according to this invention, showing two modified forms of needle seats for selective use with a barrel;
Fig. 14 is a fragmentary sectional view of the seventh preferred embodiment, showing a selected one of the needle seats retained in the barrel;
Fig. 15 is a fragmentary exploded sectional view of the eighth preferred embodiment of a disposable syringe according to this invention, showing three modified forms of needle seats for selective use with a barrel;
Fig. 16 is a fragmentary sectional view of the eighth preferred embodiment in a disposal state, showing a selected one of the needle seats retained in the barrel;
Fig. 17 is an exploded sectional view of the ninth preferred embodiment of a disposable syringe according to this invention;
Fig. 18 is a fragmentary sectional view of the ninth preferred embodiment, showing a needle seat retained in a barrel;
Fig. 19 is a partly cross-sectional view of a barrier portion taken along lines I-I of Fig. 18;
Fig. 20 is a partly cross-sectional view of a retaining portion taken along lines II-II of Fig. 18;
Fig. 21 is a view similar to Fig. 20, but showing how a needle seat is turned relative to the retainingmember to another angular position;
Fig. 22 is a sectional view of the ninth preferred embodiment in a disposal state;
Fig. 23 isafragmentarysectionalviewof the tenthpref erred embodiment of a disposable syringe according to this invention;
Fig. 24 is a fragmentary sectional view of the eleventh preferred embodiment of a disposable syringe according to this invention;
Fig. 25 is a sectional view of the eleventh preferred embodiment in a disposal state;
Fig. 26 is a fragmentary sectional view of the twelfth preferred embodiment of a disposable syringe according to this invention;
Fig. 27 is a sectional view of the twelfth preferred embodiment in a disposal state,
Fig. 28 is a fragmentary sectional view of the thirteenth preferred embodiment of a disposable syringe according to this invention;
Fig. 29 is a sectional view of the thirteenth preferred embodiment in a disposal state; and
Fig. 30 is an exploded sectional view of the fourteenth preferred embodiment of a disposable syringe according to this invention, showing three modified forms of needle assembly for selective use with a barrel.

Before the present invention is described in greater detail, it should be noted that same reference numerals have been used to denote like elements throughout the specification.

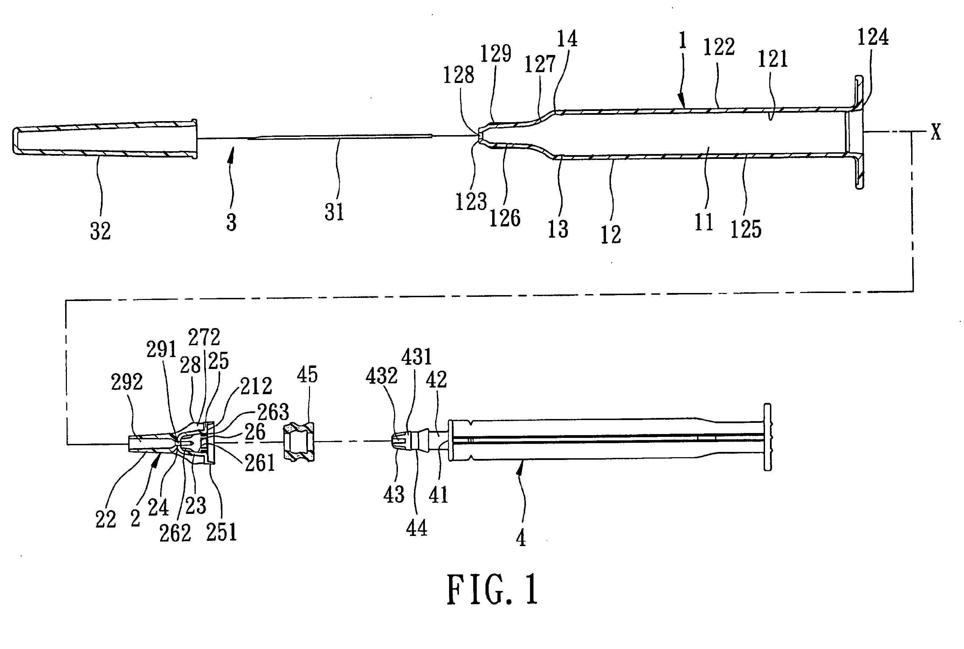

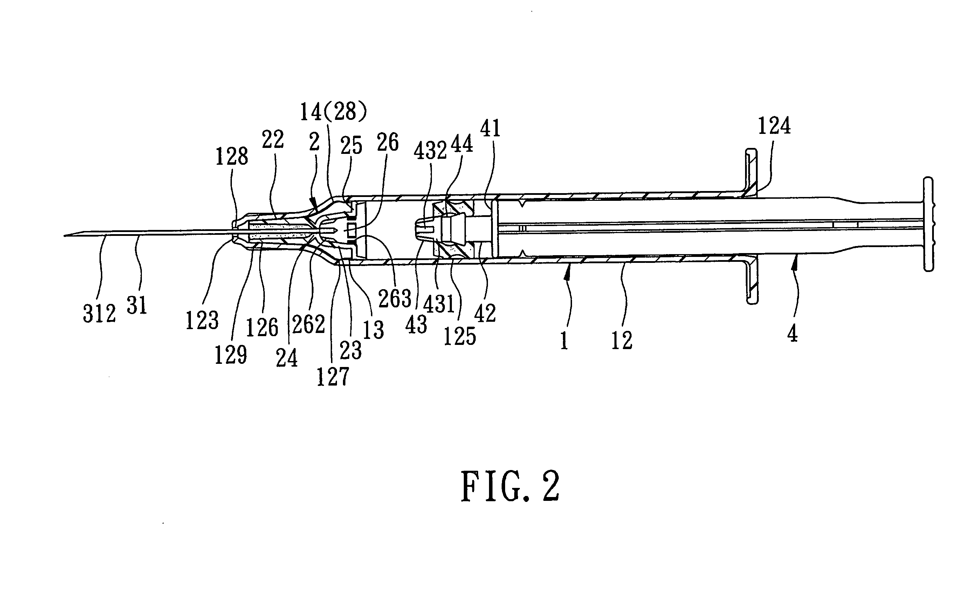

Referring to Figs. 1 to 3, the first preferred embodiment of a disposable syringe according to the present invention is shown to comprise a barrel 1, a tubular needle seat 2, a needle assembly 3 including a needle cannula 31 and a tip protector 32, and a plunger 4.

The barrel 1 has a surrounding barrel wall 12 which surrounds an axis (X) in a longitudinal direction and which defines a passage 11 therein that has front and rear open ends 123, 124 opposite to each other. The surrounding barrel wall 12 has inner and outer wall surfaces 121, 122. The inner wall surface 121 includes a smaller-diameter front surface segment 126 and a larger-diameter rear surface segment 125 proximate to the front and rear open ends 123, 124, respectively, and an intermediate surface segment 127 disposed therebetween and converging from the rear surface segment 125 to the front surface segment 126.

The intermediate surface segment 127 has a retaining region 14 which is axially rearwardly disposed from the front surface segment 126 and which extends radially and outwardly to form an annular recess 14. An annular shoulder abutment 13 is formed on the intermediate surface segment 127 rearwardly of the retaining region 14. Moreover, the intermediate surface segment 127 defines first and second friction diminishing regions 1271, 1272 which are respectively interposed between the retaining region 14 and the front surface segment 126, and between the retaining region 14 and the rear surface segment 125 to be described in greater detail hereinafter.

The outer wall surface 122 of the barrel 1 has a rib portion 129 extending in the longitudinal direction and disposed adjacent to the front open end 123. The tip protector 32 of the needle assembly 3 is disposed to sleeve on the outer wall surface 122 from the front open end 123, and is frictionally retained by the rib portion 129 for shielding the needle cannula 31.

The needle seat 2 is insertable into the passage 11 from the rear open end 124 to surround the axis (X) . The needle seat 2 includes a front engaging portion 22, a retaining portion 23, a rear engaging portion 25, a surrounding sealing flange 251, a plurality of radially yieldable catches 263, and a plurality of axially extending ribs 262.

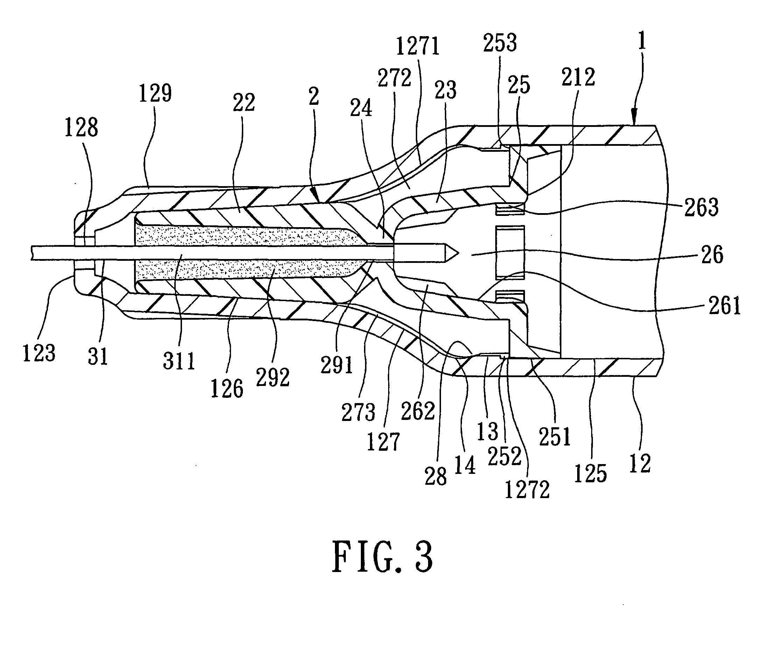

The front engaging portion 22 is configured to be in fluid-tight engagement with the front surface segment 126, and is disposed to fix the needle cannula 31 of the needle assembly 3 along the axis (X) . Specifically, the front engaging portion 22 has a filling hole 292 filled with an adhesive to affix a secured segment 311 of the needle cannula 31 to the front engaging portion 22 (see Fig. 3) . In this embodiment, the front surface segment 126 of the barrel 1 is configured to converge toward the front open end 123 to form a narrow opening 128 in the front open end 123 for passage of a sharp segment 312 of the needle cannula 31, thereby preventing removal of the needle seat 2 from the front open end 123.

The retaining portion 23 is disposed rearwardly of the front engaging portion 22, and is retained at the retaining region 14 by virtue of a first frictional force when the needle seat 2 is in a position of use. In particular, the retaining portion 23 has a plurality of fins 272 which are angularly displaced from one another about the axis (X), and which are spaced apart from the first friction diminishing region 1271 radially so as to form a first movement space 273 therebetween. Each fin 272 is configured such that a contour constituted by the fins 272 about the axis (X) serves as a protrusion 28. The protrusion 28 is retained in the annular recess 14 by virtue of the first frictional force.

The rear engaging portion 25 extends rearwardly from the retaining portion 23 to terminate at a rearwardly facing wall 212. The surrounding sealing flange 251 extends from the rearwardly facing wall 212 radially and outwardly to be in fluid-tight engagement with the rear surface segment 125, and extends forwardly to terminate at a surrounding flange surface 253. The surrounding flange surface 253 is spaced apart from the shoulder abutment 13 by the second friction diminishing region 1272 so as to forma second movement space 252 therebetween. Furthermore, the rear engaging portion 25 has an inner tubular wall surface 261 which surrounds the axis (X) to define a cavity 26 that extends from the rearwardly facing wall 212 towards the front engaging portion 22 and that terminates at a ceiling wall 24. The ceiling wall 24 has an axial hole 291 which is in fluid communication with the filling hole 292 to establish a fluid communication between the needle cannula 31 and the cavity 26.

The radiallyyieldable catches 263 and the axially extending ribs 262 are formed on the inner tubular wall surface 261 distal from and proximate to the ceiling wall 24, respectively, and are angularly displaced from one another about the axis (X) . The radially yieldable catches 263 are yieldable radially and outwardly in response to a kinetic frictional force to be described in greater detail hereinafter.

The plunger 4 is disposed to be movable in the passage 11 along the rear surface segment 125, and has a front end wall 41, an engaging head 43 which is opposite to the front end wall 41 in the longitudinal direction and which has a plurality of axially extending grooves 432, a neck 44 which is interposed between the engaging head 43 and the front end wall 41, and which is of a dimension such that a rearwardly facing shoulder wall 431 is formed between the engaging head 43 and the neck 44, and such that the aforesaid kinetic frictional force is generated as a result of axial movement of the engaging head 43 relative to the radially yieldable catches 263 towards the ceiling wall 24, and an axially extending stem 42 which is interposed between the neck 44 and the front end wall 41. A deformable sealing member 45 is sleeved on the engaging head 43 and the neck 44, is in frictional engagement with the engaging head 43 with a second frictional force, and is sealingly slidable relative to the rear surface segment 125. Furthermore, the deformable sealing member 45 is spaced apart from the front end wall 41 so as to permit relative movement of the deformable sealing member 45 towards the front end wall 41 when a pushing force is applied to the plunger 4 to overcome the second frictional force to be described in greater detail hereinafter.

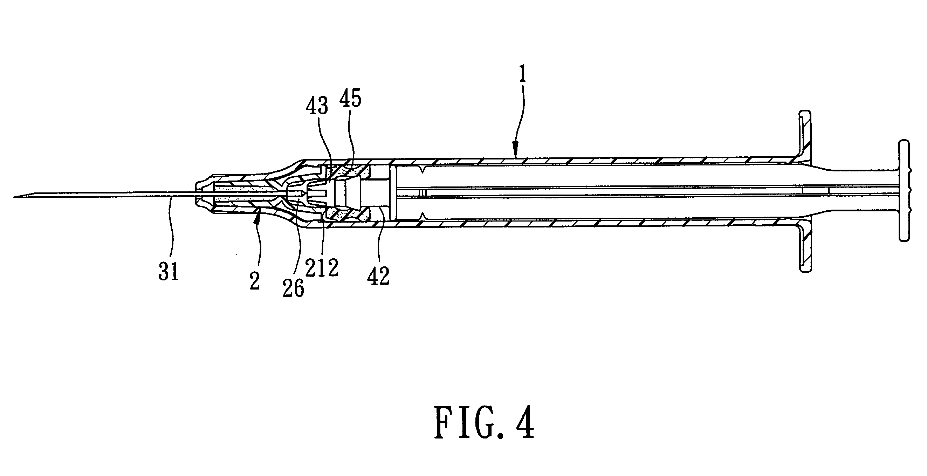

Referring to Fig. 4, in the position of use, i.e., during an injection procedure, as shown in Fig. 4, the deformable sealing member 45 is moved forward by virtue of forward movement of the plunger 4 to abut against the rearwardly facing wall 212, while the engaging head 43 extends into the cavity 26. The syringe is thus placed in an injection completed state.

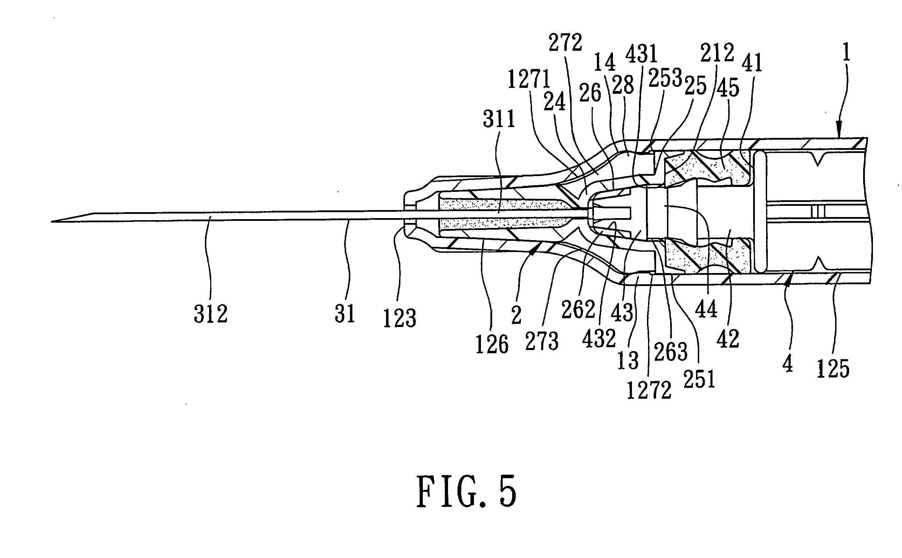

Referring to Fig. 5, when the plunger 4 is to be placed in a disposal position, the engaging head 43 is kept moving towards the ceiling wall 24 by a pushing force which is applied to the plunger 4, and which, when the deformable sealing member 45 is blocked by the rearwardly facing wall 212 from moving with the engaging head 43 and is moved rearwardly towards the front end wall 41, overcomes the second frictional force, thereby exposing the neck 44 to permit the rearwardly facing shoulder wall 431 to be forced to slip over the radiallyyieldable catches 263 and to be retained thereby such that the rearwardly facing shoulder wall 431 is prevented from moving rearwardly. Meanwhile, the axially extending grooves 432 are brought to mate with the axially extending ribs 262 so as to put the needle seat 2 into splined engagement with the engaging head 43.

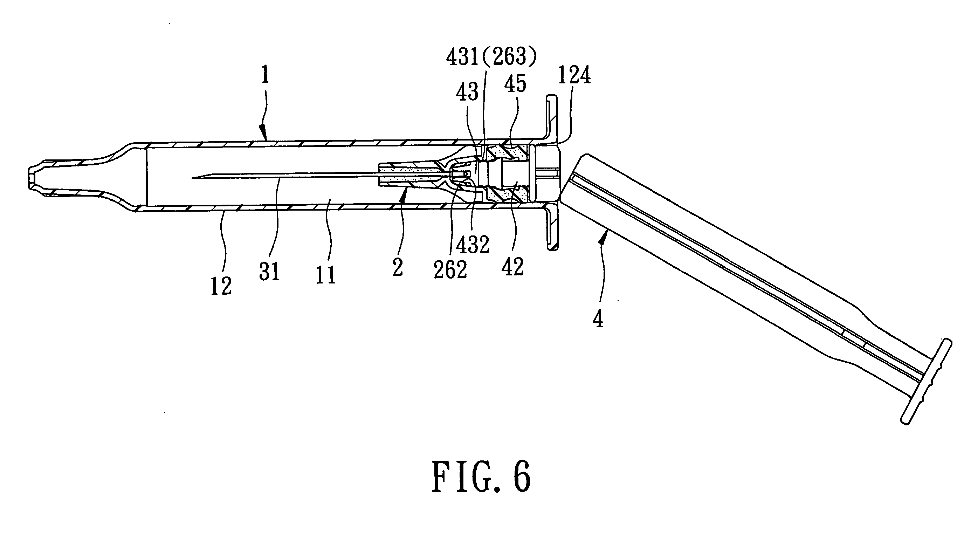

Thereafter, continued movement of the engaging head 43 towards the ceiling wall 24, against the first frictional force, i. e. the frictional retentionof the protrusion 28 in the annular recess 14, forces the fins 272 and the surrounding sealing flange 251 to move past the first and second friction diminishing regions 1271, 1272, respectively, until the surrounding flange surface 253 abuts against the shoulder abutment 13. Subsequent turning of the plunger 4 by the user to turn the needle seat 2 relative to the barrel 1 further diminish the friction therebetween to facilitate a subsequent pulling action of the plunger 4 whereby the needle seat 2 is brought towards the rear open end 124 by virtue of the retention of the rearwardly facing shoulderwall 431 by the radially yieldable catches 263, thereby retracting the needle cannula 31 into the passage 11, as shown in Fig. 6. Finally, the plunger 4 can be broken at a weakened part for convenient disposal of the syringe.

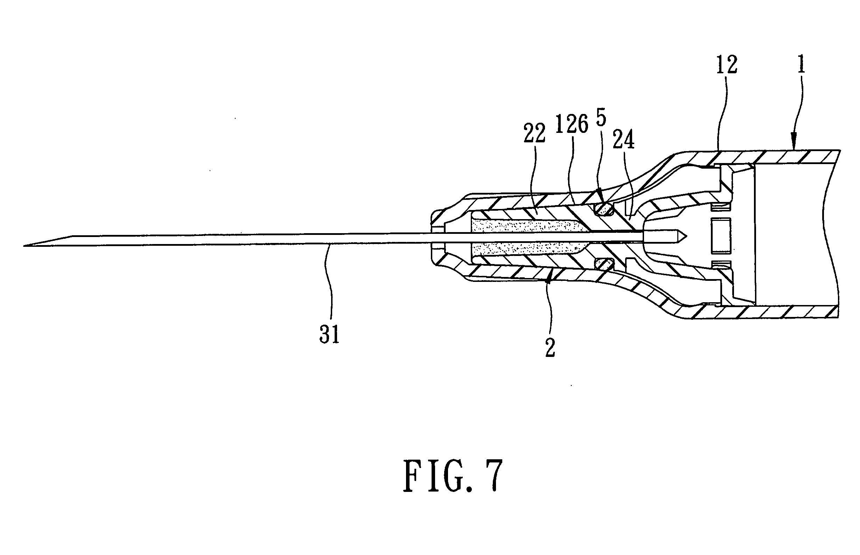

Fig. 7 shows the second preferred embodiment of a disposable syringe according to this invention. In addition to the components of the first preferred embodiment described above, the disposable syringe of this embodiment further comprises an O-ring 5 which is sleeved tightlyon the front engaging portion 22 of the needle seat 2, and which surrounds the axis (X) so as to enhance fluid-tightness of the engagement between the front surface segment 126 and the front engaging portion 22.

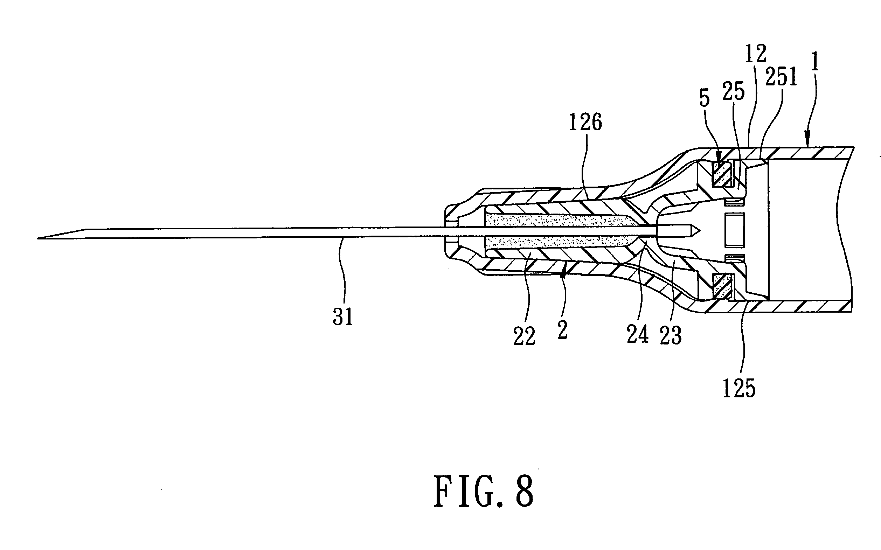

In the third preferred embodiment of a disposable syringe shown in Fig. 8, an O-ring 5 is disposed on the rear engaging portion 25 so as to enhance fluid-tightness of the engagement between the rear surface segment 125 and the surrounding sealing flange 251.

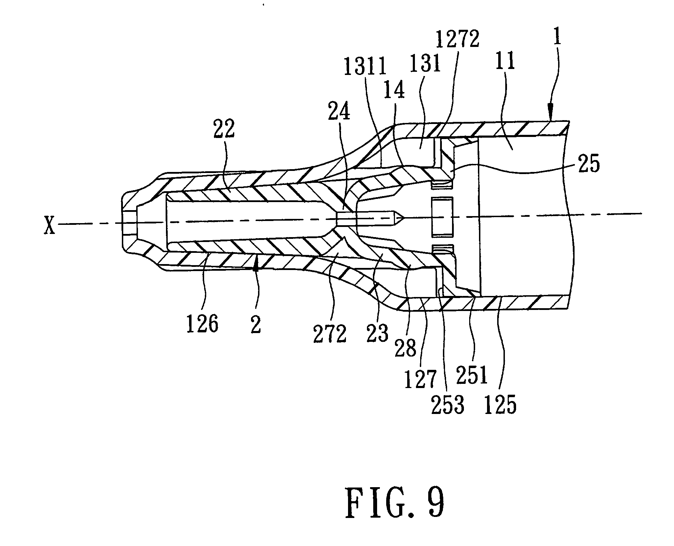

Referring to Fig. 9, the fourth preferred embodiment of a disposable syringe according to this invention is similar to the first preferred embodiment in construction. The differences reside in that the intermediate surface segment 127 has a plurality of fins 131 which are angularly displaced from one another about the axis (X) and which extend rearwardly to be spaced apart from the surrounding sealing flange 251 by the second friction diminishing region 1272. In addition, each of the fins 131 extends radially and inwardly to terminate at a distal end 1311. The distal ends 1311 of the fins 131 are configured such that a contour constituted by the distal ends 1311 about the axis (X) serves as the annular recess 14. Moreover, the retaining portion 23 is formed with an annular protrusion 28 to be retained in the recess 14 by virtue of the first frictional force. Furthermore, a plurality of fins 272 are formed on the ceiling wall 24, and are angularly displaced from one another.

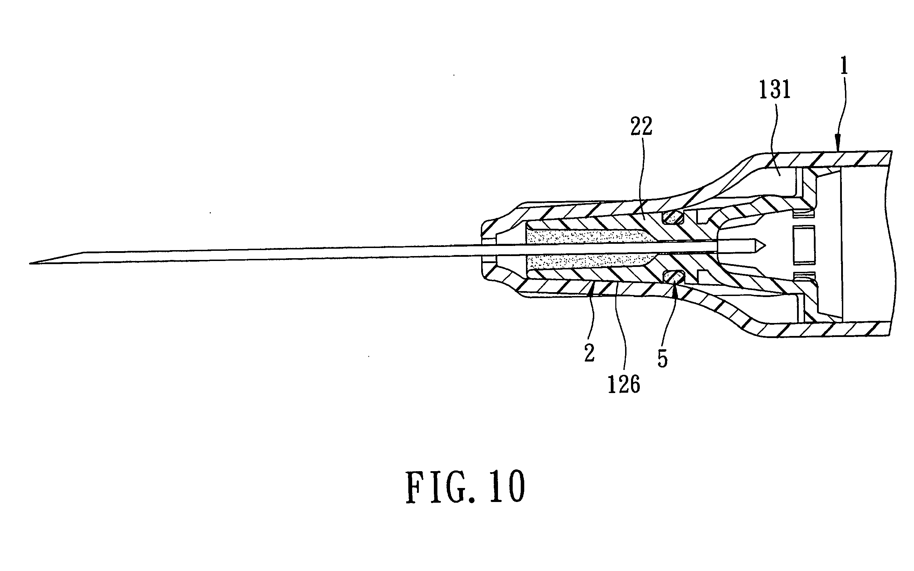

Fig. 10 shows the fifth preferred embodiment of a disposable syringe according to this invention, which is substantially similar to the fourth preferred embodiment. In this embodiment, an O-ring 5 is further sleeved tightly on the front engaging portion 22 of the needle seat 2, and is disposed to surround the axis (X) so as to enhance the fluid-tightness of the engagement between the front surface segment 126 and the front engaging portion 22.

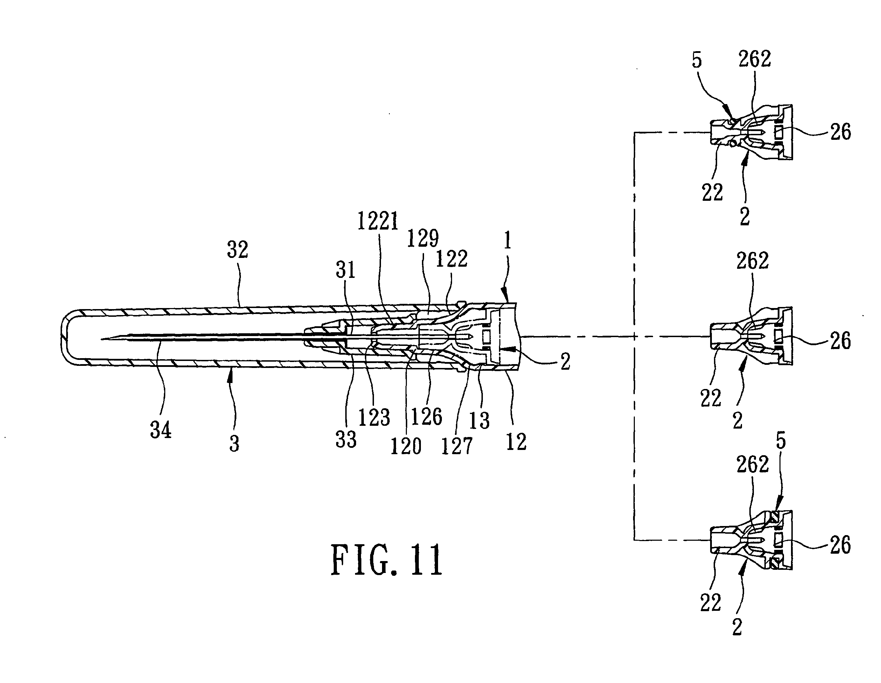

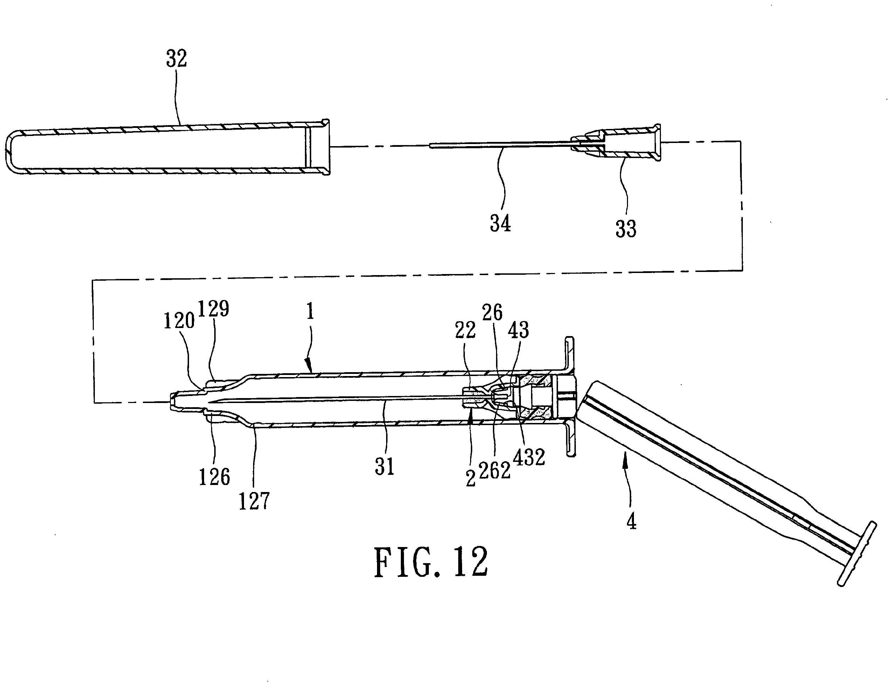

Referring to Figs. 11 to 12, the sixth preferred embodiment of a disposable syringe is shown to include a selected one of the needle seats 2 of the above embodiments. In this embodiment, the outer wall surface 122 of the barrel 1 has an annular step portion 120 that faces forwardly, and that is distal from the front open end 123, and a surrounding front segment 1221 interposed between the front open end 123 and the annular step portion 120. The ribs 129 are formed rearwardly of the surrounding front segment 1221 so as to retainingly engage the tip protector 32. In addition, the disposable syringe further comprises a catheter hub 33 and a tubular catheter 34 for performing an intravenous catheter introducing process. Specifically, the catheter hub 33 includes a surrounding hub wall whichhas a sleeve portion that is sleeved on the surrounding front segment 1221 and that has a terminal edge abutting against the step portion 120, and a tip portion opposite to the sleeve portion along the axis (X) . The tubular catheter 34 includes a proximate segment which is disposed in the tip portion and which extends along the axis (X), and a distal segment which extends from the proximate segment along the axis (X) to project outwardly of the tip portion. As such, after the tubular catheter 34 is introduced into a patient' s vein by insertion of the needle cannula 31, the barrel 1 is separated from the catheter hub 33 so as to complete the intravenous catheter introducing process. The needle cannula 31 is then retracted into the barrel 1 in the same manner as described above, as shown in Fig. 12.

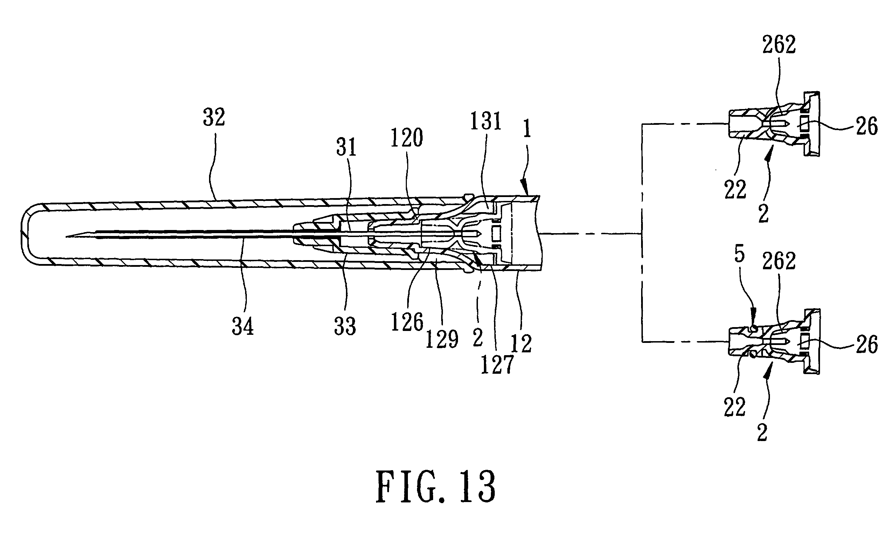

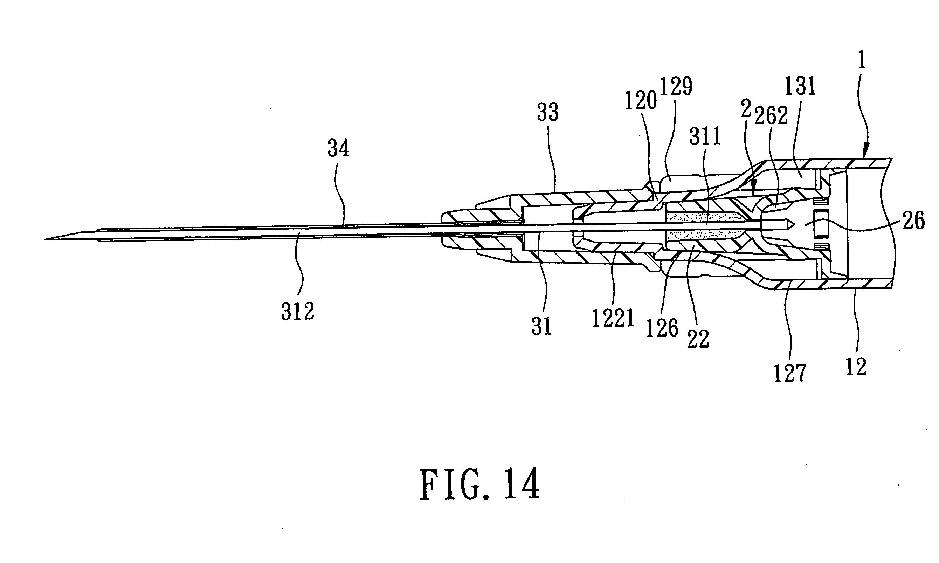

Referring to Figs. 13 and 14, the seventh preferred embodiment of a disposable syringe is shown to be similar to the sixth preferred embodiment in construction and function, except that the barrel 1 and the needle seat 2 are the same as those of the fourth and fifth preferred embodiments shown in Figs. 9 and 10. Furthermore, the front engaging portion 22 of the needle seat 2 is filled with an adhesive to affix a secured segment 311 of the needle cannula 31 to the front engagingportion 22.

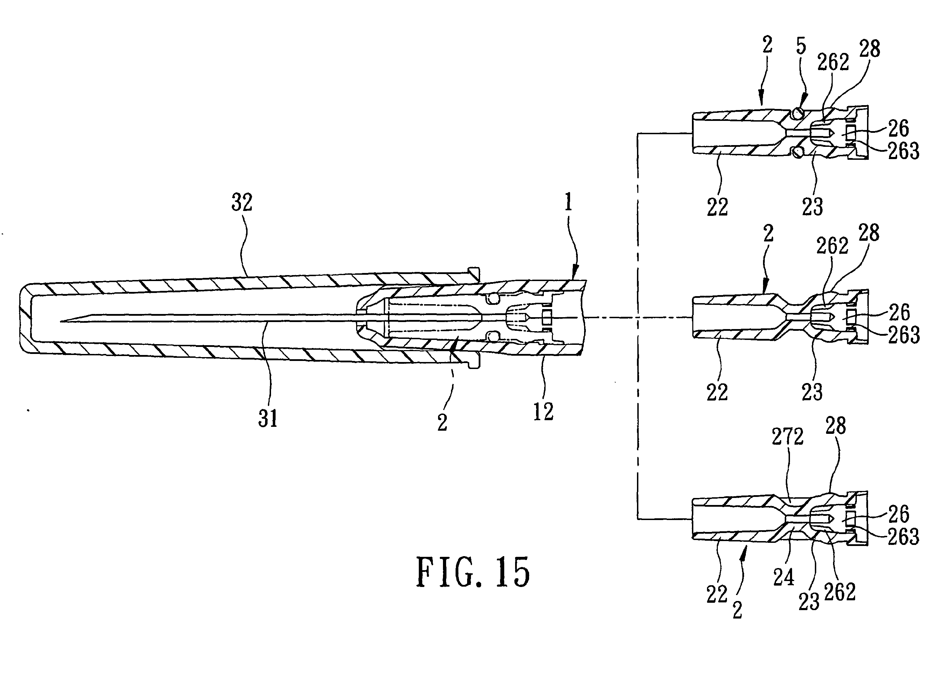

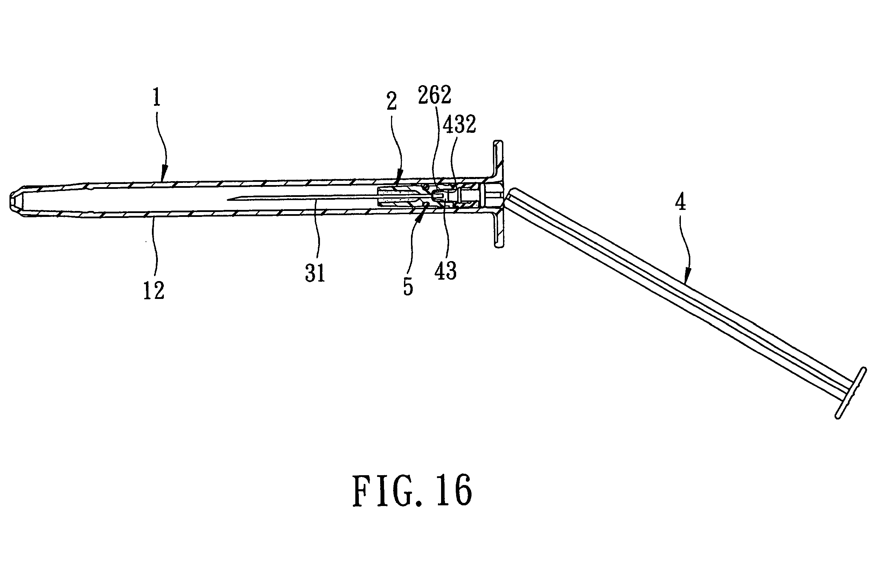

Referring to Figs. 15 and 16, the disposable syringe of the eighth preferred embodiment is adapted for injecting medication of an extremely small volume, such as 1 ml. That is, the barrel 1 and the plunger 4 have relatively smaller diameters. The needle seat 2 may have an O-ring 5 or fins 272 disposed on the ceiling wall 24.

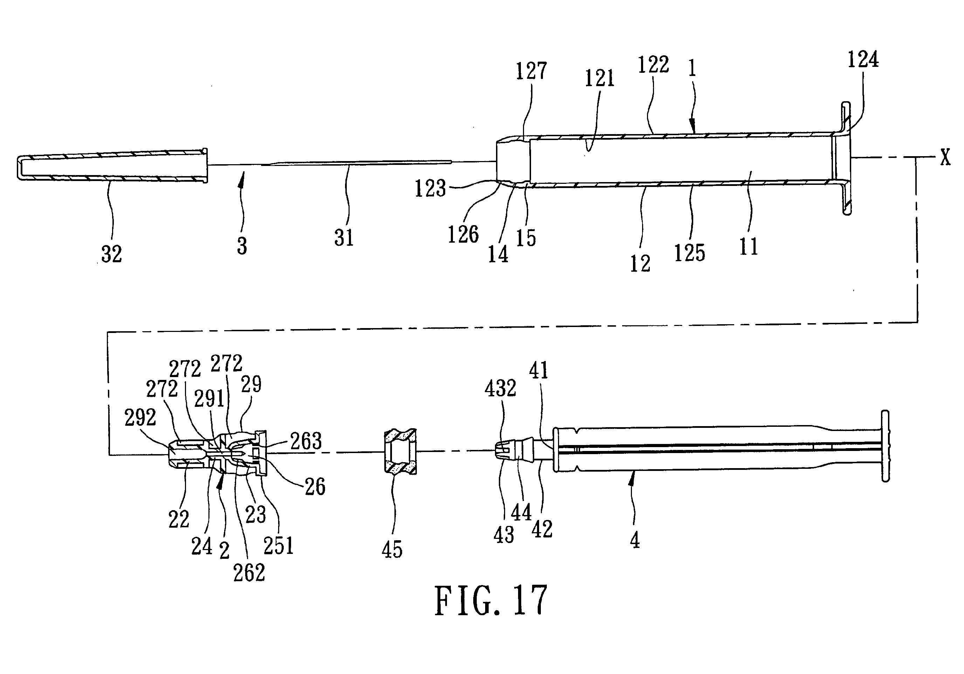

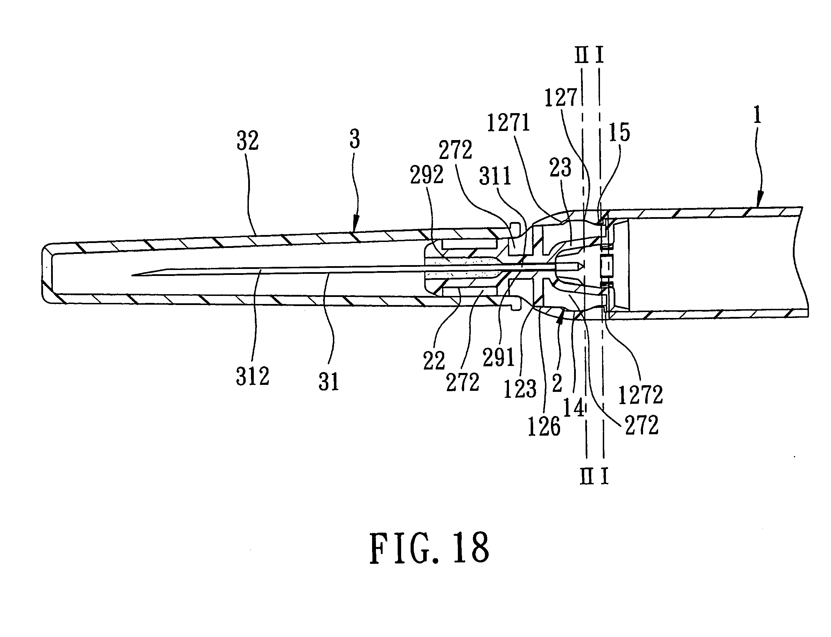

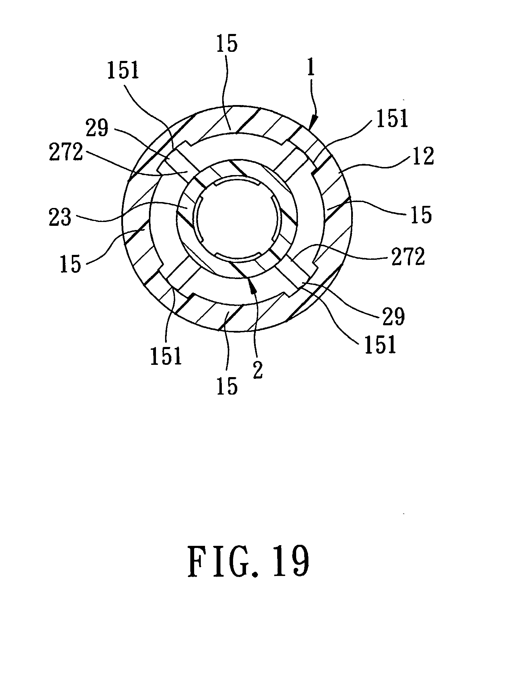

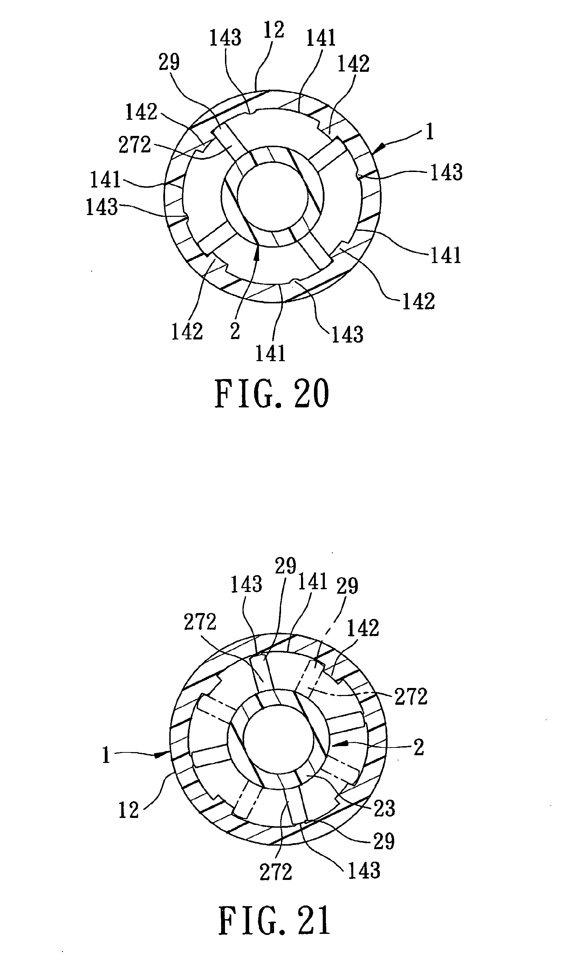

Referring to Figs. 17 and 18, the ninth preferred embodiment of a disposable syringe according to this invention is shown to be similar to the first preferred embodiment in construction. The differences therebetween are as follows. The front engaging portion 22 of the needle seat 2 has a seat segment which is configured to extend outwardly of the barrel 1 from the front open end 123 when the needle seat 2 is in the position of use. With reference to Fig. 19, the intermediate surface segment 127 has a plurality of barriers 15 which are angularly displaced from one another about the axis (X) so as to define a recessed access 151 between two adjacent ones of the barriers 15. With reference to Fig. 20, the retaining region 14 has a plurality of recesses 141 which are angularly displaced from one another about the axis (X) and which are spaced apart from one another by protrusions 142, and a plurality of bumps 143 respectively extending from the recesses 141 inwardly and radially.

The retaining portion 23 of the needle seat 2 has a plurality of partitions 272 in the form of fins 272 which are angularly displaced from one another about the axis (X), and a plurality of blocking segments 29 which respectively extend from the partitions 272 radially and outwardly. As mentioned above, when the needle seat 2 is brought to be inserted into the passage 11 from the rear open end 124 during assembly, and immediately after each of the partitions 272 is brought to pass the corresponding recessed access 151 between two adjacent ones of the barriers 15, as shown in Fig. 19, each of the partitions 272 is turned a predetermined angle in one of clockwise and counterclockwise directions such that each of the blocking segments 29 is received in and is engaged with a respective one of the recesses 141 by virtue of the first frictional force, as shown in Fig. 20, while being prevented by the corresponding barrier 15 from axial movement relative to the second diminishing region 1272, thereby placing the needle seat 2 firmly in the position of use.

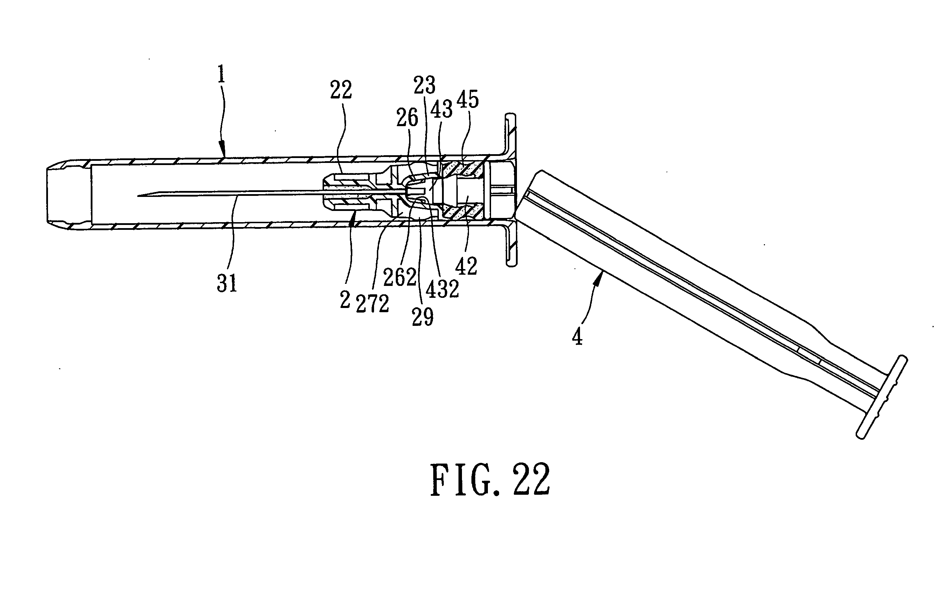

Moreover, when the plunger 4 is placed in the disposal position and the needle seat 2 is retained onto the plunger 4, the user can rotate the needle seat 2 so that the partitions 272 are turned the predetermined angle in a corresponding one of the counterclockwise and clockwise directions, as indicated by dotted lines in Fig. 21 so that the blocking segments 29 slip over the bumps 143, respectively (as indicated by solid lines in Fig. 21) . Thus, the user can be aware of the approaching of the blocking segments 29 to a position where the blocking segments 2 9 are unrestrained by the barriers 15 and are permitted to perform the axial movement. Therefore, the needle seat 2 is allowed to be pulled rearwardly so as to retract the needle cannula 31 into the passage 11, as shown in Fig. 22.

Furthermore, in addition to having the partitions 272 formed on the retaining portion 23, the needle seat 2 has a plurality of fins 272 formed on the ceiling wall 24 and on the seat segment of the front engaging portion 22. The tipprotector 32 is disposed to sleeve on and is frictionally retained onto the seat segment of the front engaging portion 22 for shielding the sharp segment 312 of the needle cannula 31.

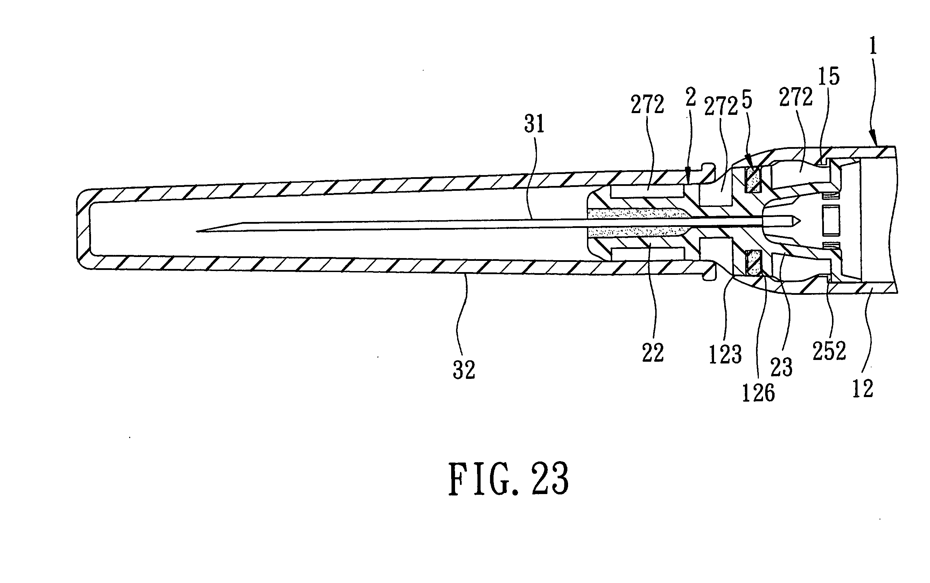

Referring to Fig. 23, the tenth preferred embodiment of a disposable syringe according to this invention is similar to the ninth preferred embodiment, and further comprises an O-ring 5 which is disposed on the front engaging portion 22 of the needle seat 2 so as to enhance fluid-tightness of the engagement between the front engaging portion 22 and the front surface segment 126 of the barrel 1.

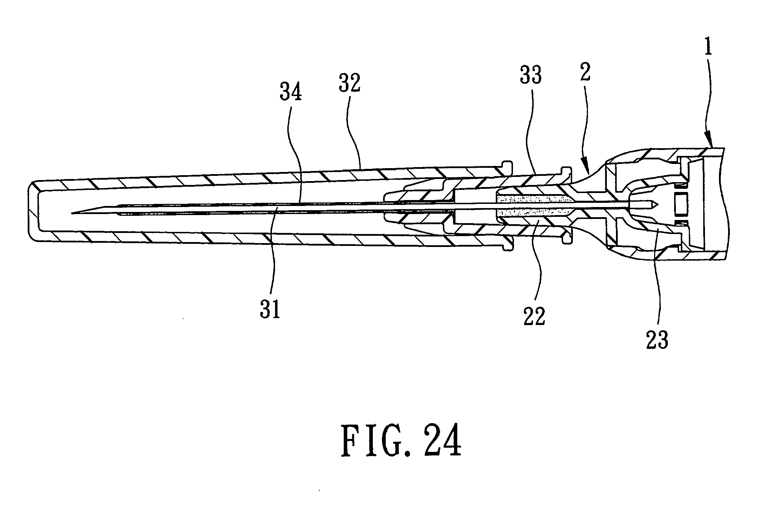

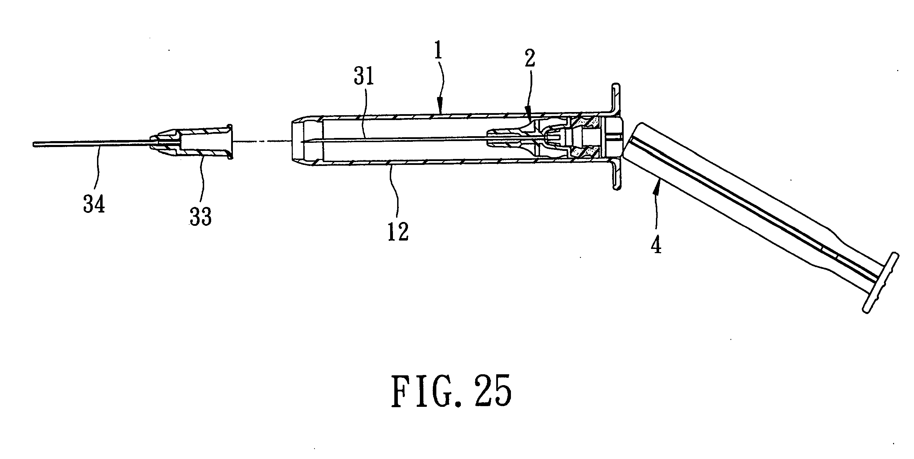

Referring to Figs. 24 and 25, in the eleventh preferred embodiment of a disposable syringe according to this invention, a catheter hub 33 and a tubular catheter 34 are further provided for performing an intravenous catheter introducing process. The catheter hub 33 includes a surrounding hub wall which has a sleeve portion that is sleeved on the seat segment of the front engaging portion 22, and a tip portion opposite to the sleeve portion along the axis (X). The tubular catheter 34 includes a proximate segment which is disposed in the tip portion and which extends along the axis (X), and a distal segment which extends from the proximate segment along the axis (X) and outwardly of the tip portion. Moreover, since the sleeve portion of the catheter hub 33 is sleeved on the seat segment of the front engaging portion 22, there is no need to form fins on the seat segment.

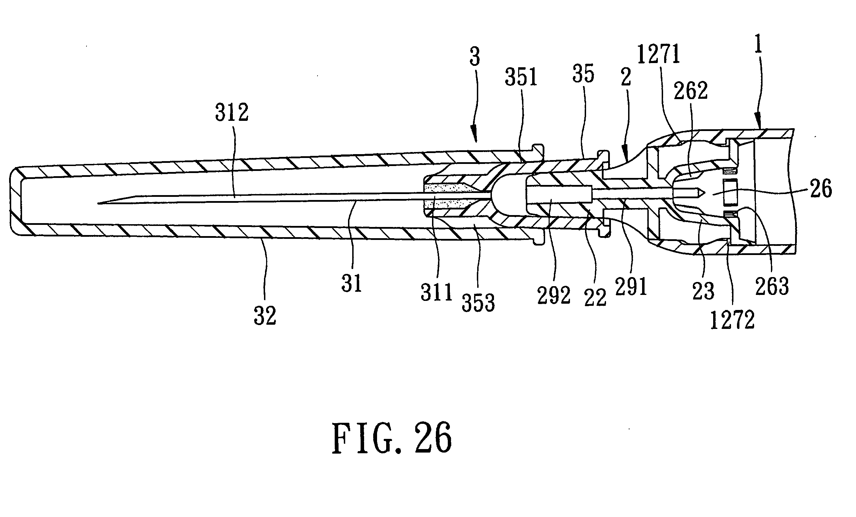

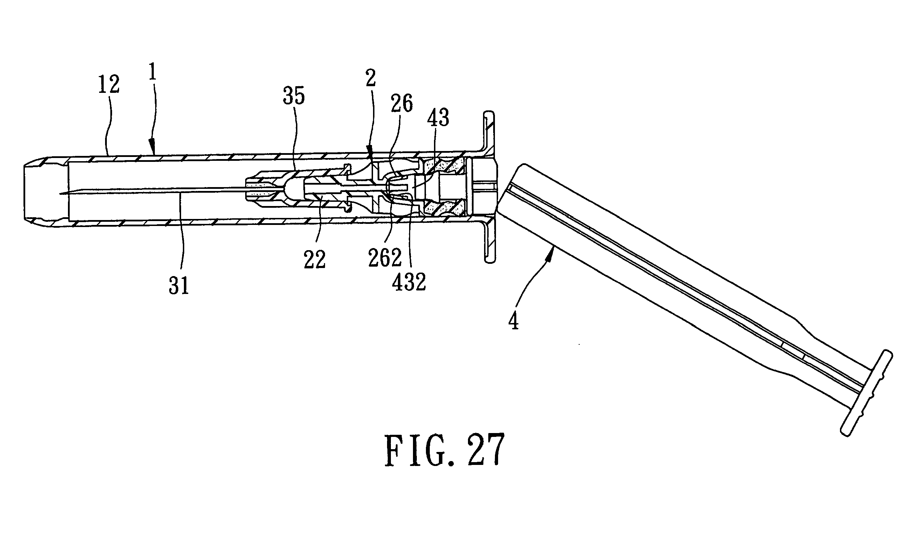

Referring to Figs. 26 and 27, the twelfth preferred embodiment of a disposable syringe according to this invention is shown to be similar to the ninth preferred embodiment. In this embodiment, the needle seat 2 further includes a hub segment 35 which is configured to fix the secured segment 311 of the needle cannula 31 along the axis (X), and which is sleeved on the seat segment of the front engaging portion 22 to establish fluid communication between the needle cannula 31 and the axial hole 291 through the filling hole 292 which is free fromadhesive . The hub segment 35 has a rib portion 353 formed on an outer surface 351 thereof so as to retain the tip protector 32 for shielding the sharp segment 312 of the needle cannula 31.

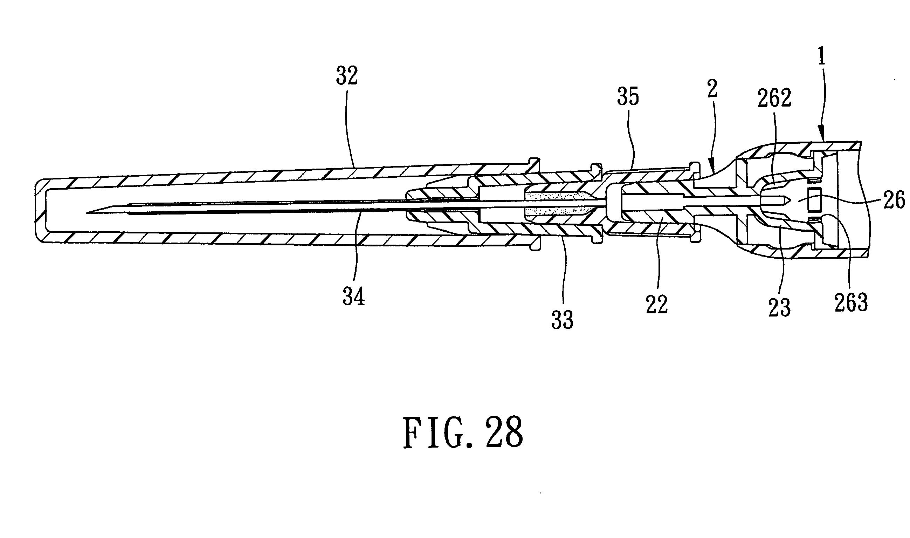

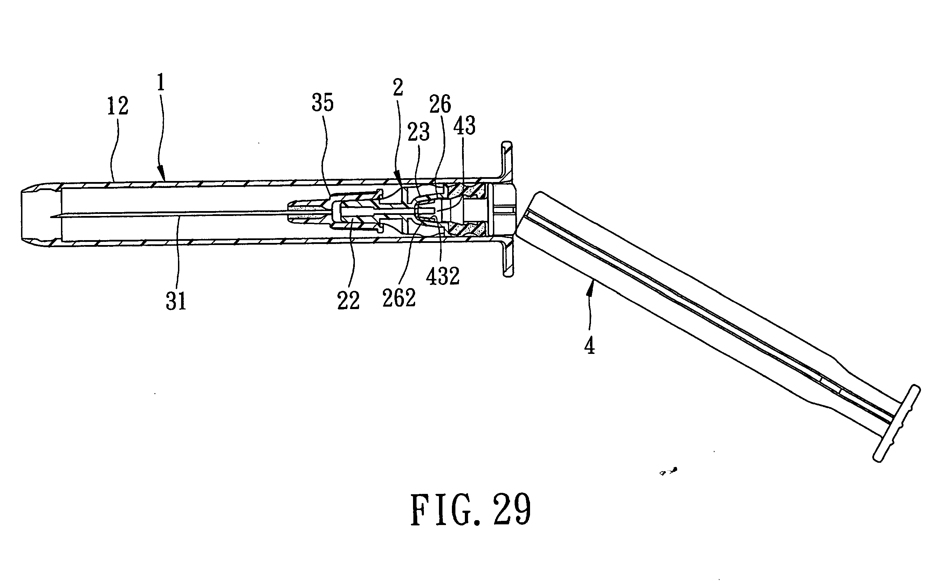

Referring to Figs. 28 and 29, the thirteenth preferred embodiment of a disposable syringe according to this invention is shown to be similar to the twelfth preferred embodiment in construction, and further comprises a catheter hub 33 and a tubular catheter 34 for performing an intravenous catheter introducing process. That is, the catheter hub 33 includes a surrounding hub wall which has a sleeve portion sleeved on the hub segment 35, and a tip portion opposite to the sleeve portion along the axis (X) . The tubular catheter 34 includes a proximate segment which is disposed in the tip portion and which extends along the axis (X), and a distal segment which extends from the proximate segment along the axis (X) and outwardly of the tip portion. The tip protector 32 is sleeved retainingly on the surrounding hub wall of the catheter hub 33.

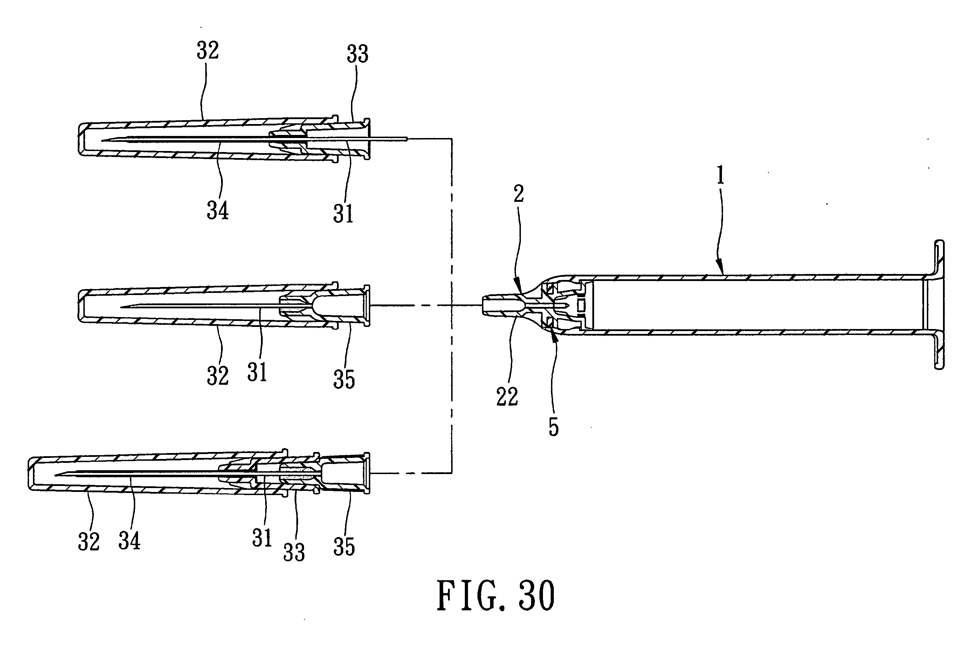

Referring to Fig. 30, in the fourteenth preferred embodiment of a disposable syringe according to this invention, which has a construction similar to that of the eleventh preferred embodiment, an O-ring 5 is additionally disposed on the front engaging portion 22 of the needle seat 2. Moreover, any forms of the needle assembles 3, such as that including the catheter hub 33 and the tubular catheter 34 , that including the hub segment 35, and that including the catheter hub 33, the tubular catheter 34 and the hub segment 35, can be provided for use with the needle seat 2 as required.

The advantages of the disposable syringe of this invention are as follows:
1. The retaining portion 23 and the surrounding sealing flange 251 are moved past the first and second friction diminishing regions 1271,1272, respectively, when the plunger 4 is to be placed in the disposal position so as to diminish the friction between the needle seat 2 and the inner wall surface 121 of the barrel 1, thereby facilitating a subsequent pulling action of the plunger 4 for retracting the needle cannula 31 into the barrel 1. Moreover, by virtue of the splined engagement between the axially extending ribs 262 and the grooves 432, the needle seat 2 is rotated with the plunger 4 to thereby further diminish the friction between the needle seat 2 and the inner wall surface 121 of the barrel 1. Therefore, the retraction of the needle cannula 31 can be successful and steady.
2. The provision of angularly displaced fins 272 on the needle seat 2 helps prevent contraction and deformation of the needle seat 2 during plastic injection molding due to the cohesion property of the plastic material, thereby reducing failure in production.
3. Since the front engaging portion 22 and the surrounding sealing flange 251 of the needle seat 2 are in fluid-tight engagement with the front and rear surface segments 126, 125 of the barrel 1, the engagement being enhanced by the provision of O-rings 5 and by virtue of the first frictional force generated between the retaining portion 23 (the protrusion 28) and the retaining region 14 (the annular recess 14), the needle seat 2 can be firmly retained in the passage 11 of the barrel 1 with a fluid-tight engagement therebetween.

## Claims

1. A disposable syringe comprising:
a barrel (1) having a surrounding barrel wall (12) which surrounds an axis (X) in a longitudinal direction and which defines a passage (11) therein that has front and rear open ends (123,124) opposite to each other, said surrounding barrel wall (12) having an inner wall surface (121) which includes front and rear surface segments (126, 125) proximate to said front and rear open ends (123,124), respectively, and an intermediate surface segment (127) disposed therebetween,
a needle cannula (31);
a tubular needle seat (2) configured to be insertable into said passage (11) from said rear open end (124), and surrounding the axis (X); and
a plunger (4) disposed to be movable in said passage (11) along said rear surface segment (125), wherein :
said intermediate surface segment (127) of said barrel (1) has a retaining region (14) which is axially rearwardly disposed from said front surface segment (126), and first and second friction diminishing regions (1271,1272) which are respectively interposed between said retaining region (14) and said front surface segment (126), and between said retaining region (14) and said rear surface segment (125);
said needle seat (2) including
a front engaging portion (22) which is configured to be in fluid-tight engagement with said front surface segment (126) and which is disposed to fix said needle cannula (31) along the axis (X),
a retaining portion (23) which is disposed rearwardly of said front engaging portion (22), and which is retained at said retaining region (14) by virtue of a first frictional force when said needle seat (2) is in a position of use,
a rear engaging portion (25) which extends rearwardly from said retaining portion (23) to terminate at a rearwardly facing wall (212),
a surrounding sealing flange (251) which extends from said rearwardly facing wall (212) radially and outwardly to be in fluid-tight engagement with said rear surface segment (125), and which extends forwardly to terminate at a surrounding flange surface (253) that is movable towards said second friction diminishing region (1272) in the longitudinal direction, said rear engaging portion (25) having an inner tubular wall surface (261) which surrounds the axis (X) to define a cavity (26) that extends from said rearwardly facing wall (212) towards said front engaging portion (22) and that terminates at a ceiling wall (24), said ceiling wall (24) having an axial hole (291) which establishes a fluid communication between said needle cannula (31) and said cavity (26), and
a radially yieldable catch (263) which is disposed on said inner tubular wall surface (261) rearwardly from said ceiling wall (24), and which is yieldable radially and outwardly in response to a kinetic frictional force; said plunger (4) having a front end wall (41), an engaging head (43) which is opposite to said front end wall (41) in the longitudinal direction, a neck (44) which is interposed between said engaging head (43) and said front end wall (41), and which is of a dimension such that a rearwardly facing shoulder wall (431) is formed between said engaging head (43) and said neck (44) , and such that the kinetic frictional force is generated as a result of axial movement of said engaging head (43) relative to said radially yieldable catch (263) towards said ceiling wall (24), and a deformable sealing member (45) which is sleeved on said engaging head (43) and said neck (44), which is in frictional engagement with said engaging head (43) with a second frictional force, and which is sealingly slidable relative to said rear surface segment (125) such that,
in the position of use, said deformable sealing member (45) is moved forward by virtue of forward movement of said plunger (4) to abut against said rearwardly facing wall (212), while said engaging head (43) is extended into said cavity (26), and such that,
when said plunger (4) is to be placed in a disposal position, said engaging head (43) is kept moving towards said ceiling wall (24) by a pushing force which is applied to said plunger (4), and which, when said deformable sealing member (45) is blocked by said rearwardly facing wall (212) from moving with said engaging head (43), overcomes the second frictional force, thereby exposing said neck (44) so as to permit said rearwardly facing shoulder wall (431) to be forced to slip over said radially yieldable catch and to be retained by said radially yieldable catch (263) such that said rearwardly facing shoulder wall (431) is prevented from moving rearwardly,
said engaging head (43) and said inner tubular wall surface (261) being configured such that, aftersaidrearwardlyfacing shoulder wall (431) has slipped over said radially yieldable catch (263), continued movement of said engaging head (43) towards said ceiling wall (24), against the first frictional force, forces said retaining portion (23) and said surrounding sealing flange (251) to move past said first and second friction diminishing regions (1271,1272), respectively, so as to facilitate a subsequent pulling action of said plunger (4) whereby said needle seat (2) is brought towards said rear open end (124) by virtue of the retention of said rearwardly facing shoulder wall (431) by said radially yieldable catch (263), thereby retracting said needle cannula (31) into said passage (11).

2. The disposable syringe of Claim 1, **characterized in that** said needle seat (2) is disposed to be in splined engagement with said engaging head (43) such that once said rearwardly facing shoulder wall (431) is retained by said radially yieldable catch (263), and once said retaining portion (23) and said surrounding sealing flange (251) are moved past said first and second friction diminishing regions (1271, 1272), respectively, said needle seat (2) is enabled to be rotated with said plunger (4) to thereby further diminish friction between said needle seat (2) and said inner wall surface (121) so as to facilitate the subsequent pulling action of said plunger (4).

3. The disposable syringe of Claim 2, **characterized in that** said needle seat (2) has a plurality of axially extending ribs (262) which are formed on said inner tubular wall surface (261) proximate to said ceiling wall (24) and which are angularly displaced from one another about the axis (X), said engaging head (43) having a plurality of axially extending grooves (432) formed to mate with said axially extending ribs (262) so as to bring said needle seat (2) into the splined engagement with said engaging head (43) .

4. The disposable syringe of Claim 1, **characterized in that** said front engaging portion (22) has a filling hole (292) in fluid communication with said axial hole (291) and filled with an adhesive to affix said needle cannula to said front engaging portion (22).

5. The disposable syringe of Claim 1, **characterized in that** said intermediate surface segment has a shoulder abutment (13) which is disposed between said retaining region (14) and said second friction diminishing region (1272) to permit abutment of said surrounding flange surface (253) thereagainst immediately after said surrounding sealing flange (251) is moved past said second friction diminishing region (1272).

6. The disposable syringe of Claim 1, **characterized in that** said retaining region (14) extending radially and outwardly to form an annular recess (14), said needle seat (2) having a protrusion (28) formed on said retaining portion (23) so as to be retained in said recess (14) by virtue of the first frictional force.

7. The disposable syringe of Claim 6, **characterized in that** said retaining portion (23) has a plurality of fins (272) which are angularly displaced from one another about the axis (X) and which are spaced apart from said first friction diminishing region radially, each of said fins (272) being configured such that a contour constituted by said fins (272) about the axis (X) serves as said protrusion (28).

8. The disposable syringe of Claim 6, **characterized in that** said intermediate surface segment (127) has a plurality of fins (131) which are angularly displaced from one another about the axis (X) and which extend rearwardly to be spaced apart from said surrounding sealing flange (251) by said second friction diminishing region (1272), each of said fins (131) extending radially and inwardly to terminate at a distal end (1311), said distal ends (1311) being configured such that a contour constituted by said distal ends (1311) about the axis (X) serves as said recess (14) .

9. The disposable syringe of Claim 1, **characterized in that** said front surface segment (126) is configured to converge toward said front open end (123) to prevent removal of said needle seat (2) from said front open end (123).

10. The disposable syringe of Claim 1, **characterized in that** said surrounding barrel wall (12) has an outer wall surface (122) which has a rib portion (129) extending in the longitudinal direction and disposed adjacent to said front open end (123), said disposable syringe further comprising a tip protector (32) which is disposed to sleeve on said outerwall surface (122), and which is frictionallyretained by said rib portion (129) for shielding said needle cannula (31).

11. The disposable syringe of Claim 1, further **characterized by** an O-ring (5) which is disposed on said needle seat (2), which surrounds the axis (X), and which is configured to enhance fluid-tightness of the engagement between said front surf ace segment (126) and said front engaging portion (22), or between said rear surface segment (125) and said surrounding sealing flange (251).

12. The disposable syringe of Claim 1, **characterized in that** said surrounding barrel wall (12) has an outer wall surface (122) which has an annular step portion (120) that faces forwardly, and that is distal from said front open end (123), and a surrounding front segment (1221) interposed between said front open end (123) and said annular step portion (120), said disposable syringe further comprising
a catheter hub (33) including a surrounding hub wall which has a sleeve portion that is sleeved on said surrounding front segment (1221) and that has a terminal edge abutting against said step portion (120), and a tip portion opposite to said sleeve portion along the axis (X); and
a tubular catheter (34) including a proximate segment which is disposed in said tip portion and which extends along the axis (X), and a distal segment which extends from said proximate segment along the axis (X) to project outwardly of said tip portion.

13. The disposable syringe of Claim 1, **characterized in that** said front engaging portion (22) has a seat segment which is configured to extend outwardly of said barrel (1) from said front open end (123) when said needle seat (2) is in the position of use.

14. The disposable syringe of Claim 13, further **characterized by** a tip protector (32) which is disposed to sleeve on and which is frictionally retained to said seat segment of said front engaging portion (22) for shielding of said needle cannula (31).

15. The disposable syringe of Claim 13, **characterized in that** said front engaging portion (22) further has a hub segment (35) which is configured to fix said needle cannula along the axis (X), and which is sleeved on said seat segment to establish fluid communication between said needle cannula (31) and said axial hole (291).

16. The disposable syringe of Claim 15, further **characterized by**:
a catheter hub (33) including a surrounding hub wall which has a sleeve portion that is sleeved on said hub segment (35), and a tip portion opposite to said sleeve portion along the axis (X) ; and
a tubular catheter (34) including a proximate segment which is disposed in said tip portion and which extends along the axis (X), and a distal segment which extends from said proximate segment along the axis (X) and outwardly of said tip portion.

17. The disposable syringe of Claim 13, **characterized in that** said retaining region (14) has a plurality of recesses (141) which are angularly displaced from one another about the axis (X),
said intermediate surface segment (127) further having a plurality of barriers (15) which are angularly displaced from one another about the axis (X),
said retaining portion (23) of said needle seat (2) having a plurality of partitions (272) which are angularly displaced from one another about the axis (X), and a plurality of blocking segments (29) which respectively extend from said partitions (272) radially and outwardly, each of said blocking segments (29) being configured such that when said needle seat (2) is brought to be inserted into said passage (11) from said rear open end (124), and immediately after each of said partitions (272) is brought to pass between two adjacent ones of said barriers (15), each of said partitions (272) is turned a predetermined angle in one of clockwise and counterclockwise directions such that each of said blocking segments (28) is received in and is engaged with a respective one of said recesses (141) by virtue of the first frictional force, while being prevented by one of said two adjacent ones of said barriers (15) from axial movement relative to said second diminishing region (252), thereby placing said needle seat (2) firmly in the position of use.

18. The disposable syringe of Claim 17, **characterized in that** said retaining region (14) has a plurality of bumps (143) respectively extending from said recesses (141) inwardly and radially such that when said partitions (272) are turned the predetermined angle in a corresponding one of the counterclockwise and clockwise directions, said blocking segments (29) slip over said bumps (143) respectively so that the user is aware of movement of said blocking segments (29) to a position where said blocking segments (29) are unrestrained by said barriers (15) and are permitted to perform axial movement.

19. The disposable syringe of Claim 1, **characterized in that** said deformable sealing member (45) is disposed to be spaced apart from said front end wall (41) so as to permit relative movement of said deformable sealing member (45) towards said front end wall (41) when the pushing force is applied to overcome the second frictional force.

## Patentansprüche

1. Einwegspritze, die Folgendes umfasst:
einen Zylinder (1) mit einer umrandenden Zylinderwand (12), die eine Achse (X) in einer Längsrichtung umgibt und die einen Durchgang (11) darin definiert, der ein vorderes und
ein hinteres offenes Ende (123, 124) hat, die einander entgegengesetzt sind, wobei die umrandende Zylinderwand (12) eine Innenwandfläche (121) hat, die vordere und hintere Oberflächensegmente (126, 125) nahe dem vorderen bzw. hinteren offenen Ende (123, 124) und ein dazwischen angeordnetes Oberflächenzwischensegment (127) aufweist,
eine Hohlnadel (31);
einen röhrenförmigen Nadelsitz (2), der von dem hinteren offenen Ende (124) her
in den Durchgang (11) einsetzbar konfiguriert ist und die Achse (X) umgibt; und
einen Kolben (4), der in dem Durchgang (11) entlang dem hinteren Oberflächensegment (125) bewegbar angeordnet ist; wobei:
das Oberflächenzwischensegment (127) des Zylinders (1) eine Halteregion (14), die axial rückwärts von dem vorderen Oberflächensegment (126) angeordnet ist, und eine erste und eine zweite Reibungsminderungsregion (1271, 1272) hat, die zwischen der Halteregion (14) und dem vorderen Oberflächensegment (126) bzw. zwischen der Halteregion (14) und dem hinteren Oberflächensegment (125) angeordnet sind;
der Nadelsitz (2) Folgendes aufweist:
einen vorderen Eingriffsabschnitt (22), der für fluiddichten Eingriff mit dem vorderen Oberflächensegment (126) konfiguriert ist und zum Befestigen der Hohlnadel (31) entlang der Achse (X) angeordnet ist,
einen Halteabschnitt (23), der rückwärts von dem vorderen Eingriffsabschnitt (22) angeordnet ist und der aufgrund einer ersten Reibungskraft an der Halteregion (14) gehalten wird, wenn der Nadelsitz (2) in einer Gebrauchsposition ist,
einen hinteren Eingriffsabschnitt (25), der sich rückwärts ab dem Halteabschnitt (23) erstreckt, um an einer nach hinten gekehrten Wand (212) zu enden,
einen umrandenden Dichtungsflansch (251), der sich ab der nach hinten gekehrten Wand (212) radial und nach außen erstreckt, um in fluiddichtem Eingriff mit dem hinteren Oberflächensegment (125) zu sein, und nach vorn verläuft, um an einer umrandenden Flanschfläche (253) zu enden, die in Richtung auf die zweite Reibungsminderungsregion (1272) in der Längsrichtung bewegbar ist, wobei der hintere Eingriffsabschnitt (25) eine innere röhrenförmige Wandfläche (261) hat, die die Achse (X) umgibt, zum Definieren eines Hohlraums (26), der von der nach hinten gekehrten Wand (212) in Richtung auf den vorderen Eingriffsabschnitt (22) verläuft und an einer Deckenwand (24) endet, wobei die Deckenwand (24) ein axiales Loch (291) hat, das eine Fluidkommunikation zwischen der Hohlnadel (31) und dem Hohlraum (26) herstellt, und
ein radial nachgiebiges Schnappelement (263), das an der inneren röhrenförmigen Wandfläche (261) rückwärts von der Deckenwand (24) angeordnet ist und das als Reaktion auf eine kinetische Reibungskraft radial und nach außen nachgiebig ist;
der Kolben (4) eine vordere Endwand (41), einen Eingriffskopf (43), der der vorderen Endwand (41) in der Längsrichtung entgegengesetzt ist, einen Hals (44), der zwischen dem Eingriffskopf (43) und der vorderen Endwand (41) angeordnet ist und eine solche Abmessung hat, dass zwischen dem Eingriffskopf (43) und dem Hals (44) eine nach hinten gekehrte Schulterwandung (431) gebildet wird und dass die kinetische Reibungskraft infolge einer axialen Bewegung des Eingriffkopfs (43) relativ zum radial nachgiebigen Schnappelement (263) in Richtung auf die Deckenwand (24) erzeugt wird, und ein verformbares Dichtungselement (45) hat, das auf den Eingriffskopf (43) und den Hals (44) aufgeschoben ist, das mit einer zweiten Reibungskraft mit dem Eingriffskopf (43) in Reibungseingriff ist und das relativ zu dem hinteren Oberflächensegment (125) dichtend gleitfähig ist, so dass
in der Gebrauchsposition das verformbare Dichtungselement (45) aufgrund einer Vorwärtsbewegung des Kolbens (4) vorwärtsbewegt wird, um an der nach hinten gekehrten Wand (212) in Anlage zu kommen, während der Eingriffskopf (43) in den Hohlraum (26) ausgefahren wird, und so dass,
wenn der Kolben (4) in eine Entsorgungsstellung zu bringen ist, der Eingriffskopf (43) von einer Schubkraft in Richtung auf die Deckenwand (24) weiterbewegt wird, die auf den Kolben (4) ausgeübt wird und die, wenn das verformbare Dichtungselement (45) von der nach hinten gekehrten Wand (212) am Bewegen mit dem Eingriffskopf (43) gehindert wird, die zweite Reibungskraft überwindet, wodurch der Hals (44) freigelegt wird, um zuzulassen, dass die nach hinten gekehrte Schulterwandung (431) gezwungen wird, über das radial nachgiebige Schnappelement hinweg zu gleiten und von dem radial nachgiebigen Schnappelement (263) so festgehalten zu werden, dass die nach hinten gekehrte Schulterwandung (431) am Rückwärtsbewegen gehindert wird,
der Eingriffskopf (43) und die innere röhrenförmige Wandfläche (261) so konfiguriert sind, dass, nachdem die nach hinten gekehrte Schulterwandung (431) über das radial nachgiebige Schnappelement (263) geglitten ist, ein Weiterbewegen des Eingriffskopfs (43) in Richtung auf die Deckenwand (24) gegen die erste Reibungskraft den Halteabschnitt (23) und den umrandenden Dichtungsflansch (251) zum Vorbeibewegen an der ersten bzw. zweiten Reibungsminderungsregion (1271, 1272) zwingt, um eine nachfolgende Ziehwirkung des Kolbens (4) zu erleichtern, wodurch der Nadelsitz (2) aufgrund der Festhaltung der rückwärts gekehrten Schulterwandung (431) durch das radial nachgiebige Schnappelement (263) in Richtung auf das hintere offene Ende (124) gebracht wird, wodurch die Hohlnadel (31) in den Durchgang (11) zurückgezogen wird.

2. Einwegspritze nach Anspruch 1 **dadurch gekennzeichnet, dass** der Nadelsitz (2) so angeordnet ist, dass er mit dem Eingriffskopf (43) in verzahntem Eingriff ist, so dass, wenn die nach hinten gekehrte Schulterwandung (431) von dem radial nachgiebigen Schnappelement (263) festgehalten wird und wenn der Halteabschnitt (23) und der umrandende Dichtungsflansch (251) an der ersten bzw. zweiten Reibungsminderungsregion (1271, 1272) vorbeibewegt worden sind, der Nadelsitz (2) mit dem Kolben (4) gedreht werden kann, um **dadurch** Reibung zwischen dem Nadelsitz (2) und der Innenwandfläche (121) weiter zu vermindern, um die nachfolgende Ziehwirkung des Kolbens (4) zu erleichtern.

3. Einwegspritze nach Anspruch 2, **dadurch gekennzeichnet, dass** der Nadelsitz (2) eine Mehrzahl von axial verlaufenden Rippen (262) hat, die an der inneren röhrenförmigen Wandfläche (261) nahe der Deckenwand (24) ausgebildet sind und die winklig voneinander versetzt um eine Achse (X) angeordnet sind, der Eingriffskopf (43) eine Mehrzahl von axial verlaufenden Nuten (432) hat, die zum Zusammenpassen mit den axial verlaufenden Rippen (262) ausgebildet sind, um den Nadelsitz (2) in den verzahnten Eingriff mit dem Eingriffskopf (42) zu bringen.

4. Einwegspritze nach Anspruch 1, **dadurch gekennzeichnet, dass** der vordere Eingriffsabschnitt (22) ein Füllloch (292) hat, das mit dem axialen Loch (291) in Fluidkommunikation steht und mit einem Klebstoff zum Befestigen der Hohlnadel an dem vorderen Eingriffsabschnitt (22) gefüllt ist.

5. Einwegspritze nach Anspruch 1, **dadurch gekennzeichnet, dass** das Oberflächenzwischensegment ein Schulterwiderlager (13) hat, das zwischen der Halteregion (14) und der zweiten Reibungsminderungsregion (1272) angeordnet ist, damit die umrandende Flanschfläche (253), unmittelbar nachdem der umrandende Dichtungsflansch (251) an der zweiten Reibungsminderungsregion (1272) vorbeibewegt worden ist, daran in Anlage kommen kann.

6. Einwegspritze nach Anspruch 1, **dadurch gekennzeichnet, dass** die Halteregion (14) radial und nach außen verläuft, um eine ringförmige Aussparung (14) zu bilden, wobei der Nadelsitz (2) einen an dem Halteabschnitt (23) ausgebildeten Vorsprung (28) hat, um aufgrund der ersten Reibungskraft in der Aussparung (14) gehalten zu werden.

7. Einwegspritze nach Anspruch 6, **dadurch gekennzeichnet, dass** der Halteabschnitt (23) eine Mehrzahl von Stegen (272) hat, die winklig voneinander versetzt um die Achse (X) angeordnet sind und die von der ersten Reibungsminderungsregion radial beabstandet sind, wobei die Stege (272) jeweils so konfiguriert sind, dass eine von den Stegen (272) um die Achse (X) gebildete Kontur als der genannte Vorsprung (28) dient.

8. Einwegspritze nach Anspruch 6, **dadurch gekennzeichnet, dass** das Oberflächenzwischensegment (127) eine Mehrzahl von Stegen (131) hat, die winklig voneinander versetzt um die Achse (X) angeordnet sind und die sich rückwärts erstrecken, um durch die zweite Reibungsminderungsregion (1272) von dem umrandenden Dichtungsflansch (251) beabstandet zu sein, wobei die Stege (131) jeweils radial und einwärts verlaufen, um an einem distalen Ende (1311) zu enden, wobei die distalen Enden (1311) so konfiguriert sind, dass eine von den distalen Enden (1311) um die Achse (X) gebildete Kontur als der genannte Vorsprung (14) dient.

9. Einwegspritze nach Anspruch 1, **dadurch gekennzeichnet, dass** das vordere Oberflächensegment (126) so konfiguriert ist, dass es in Richtung auf das vordere offene Ende (123) konvergiert, um das Entfernen des Nadelsitzes (2) aus dem vorderen offenen Ende (123) zu verhindern.

10. Einwegspritze nach Anspruch 1, **dadurch gekennzeichnet, dass** die umrandende Zylinderwand (12) eine Außenwandfläche (122) hat, die einen Rippenabschnitt (129) hat, der in der Längsrichtung verläuft und neben dem vorderen offenen Ende (123) angeordnet ist, wobei die Einwegspritze ferner einen Spitzenschutz (32) umfasst, der zum aufgeschobenen Sitzen auf der Außenwandfläche (122) angeordnet ist und reibschlüssig von dem Rippenabschnitt (129) festgehalten wird zum Abschirmen der Hohlnadel (31).

11. Einwegspritze nach Anspruch 1, ferner **gekennzeichnet durch** einen O-Ring (5), der an dem Nadelsitz (2) angeordnet ist, der die Achse (X) umgibt und der zum Verbessern der Fluiddichtheit des Eingriffs zwischen dem vorderen Oberflächensegment (126) und dem vorderen Eingriffsabschnitt (22) oder zwischen dem hinteren Oberflächensegment (125) und dem umrandenden Dichtungsflansch (251) konfiguriert ist.

12. Einwegspritze nach Anspruch 1, **dadurch gekennzeichnet, dass** die umrandende Zylinderwand (12) eine Außenwandfläche (122), die einen ringförmigen Stufenabschnitt (120) hat, der nach vorn gekehrt ist und der von dem vorderen offenen Ende (123) distal ist, und ein umrandendes vorderes Segment (1221) hat, das zwischen dem vorderen offenen Ende (123) und dem ringförmigen Stufenabschnitt (120) angeordnet ist, wobei die Einwegspritze ferner Folgendes umfasst:
einen Katheteransatz (33) mit einer umrandenden Ansatzwand, die einen Muffenabschnitt hat, der auf das umrandende vordere Segment (1221) aufgeschoben ist und einen Endrand, der am Stufenabschnitt (120) in Anlage ist, und einen Spitzenabschnitt aufweist, der dem Muffenabschnitt entlang der Achse (X) entgegengesetzt ist; und
einen röhrenförmigen Katheter (34), der ein proximales Segment, das in dem Spitzenabschnitt angeordnet ist und entlang der Achse (X) verläuft, und ein distales Segment aufweist, das von dem proximalen Segment entlang der Achse (X) verläuft, um nach außen aus dem Spitzenabschnitt herauszuragen.

13. Einwegspritze nach Anspruch 1, **dadurch gekennzeichnet, dass** der vordere-Eingriffsabschnitt (22) ein Sitzsegment hat, das so konfiguriert ist, dass es sich außerhalb des Zylinders (1) von dem vorderen offenen Ende (123) erstreckt, wenn der Nadelsitz (2) in der Gebrauchsposition ist.

14. Einwegspritze nach Anspruch 13, ferner **gekennzeichnet durch** einen Spitzenschutz (32), der zum aufgeschobenen Sitzen angeordnet ist und reibschlüssig von dem Sitzsegment des vorderen Eingriffsabschnitts (22) festgehalten wird zum Abschirmen der Hohlnadel (31).

15. Einwegspritze nach Anspruch 13, **dadurch gekennzeichnet, dass** der vordere Eingriffsabschnitt (22) ferner ein Ansatzsegment (35) hat, das zum Befestigen der Hohlnadel entlang der Achse (X) konfiguriert ist und das auf das Sitzsegment aufgeschoben ist zum Herstellen einer Fluidkommunikation zwischen der Hohlnadel (31) und dem axialen Loch (291).

16. Einwegspritze nach Anspruch 15, ferner **gekennzeichnet durch**:
einen Katheteransatz (33), der eine umrandende Ansatzwand, die einen Muffenabschnitt hat, der auf das Ansatzsegment (35) aufgeschoben ist, und einen Spitzenabschnitt aufweist, der dem Muffenabschnitt entlang der Achse (X) entgegengesetzt ist; und
einen röhrenförmigen Katheter (34), der ein proximales Segment, das in dem Spitzenabschnitt angeordnet ist und entlang der Achse (X) verläuft, und ein distales Segment aufweist, das von dem proximalen Segment entlang der Achse (X) verläuft, um nach außen aus dem Spitzenabschnitt herauszuragen.

17. Einwegspritze nach Anspruch 13, **dadurch gekennzeichnet, dass** die Halteregion (14) eine Mehrzahl von Aussparungen (141) hat, die winklig voneinander versetzt um die Achse (X) angeordnet sind,
das Oberflächenzwischensegment (127) ferner eine Mehrzahl von Sperren (15) hat, die winklig voneinander versetzt um die Achse (X) angeordnet sind,
der Halteabschnitt (23) des Nadelsitzes (2) eine Mehrzahl von Trennwänden (272), die winklig voneinander versetzt um die Achse (X) angeordnet sind, und eine Mehrzahl von Sperrsegmenten (29) hat, die jeweils von den Trennwänden (272) radial und nach außen verlaufen, wobei jedes der Sperrsegmente (29) so konfiguriert ist, dass jede der Trennwände (272), wenn der Nadelsitz (2) zum Einsetzen in den Durchgang (11) vom hinteren offenen Ende (124) her gebracht wird und unmittelbar nachdem jede der Trennwände (272) zum Hindurchführen zwischen zwei benachbarten der Sperren (15) gebracht worden ist, um einen vorbestimmten Winkel im Uhrzeigersinn oder entgegen dem Uhrzeigersinn gedreht wird, so dass jedes der Sperrsegmente (28) aufgrund der ersten Reibungskraft in einer jeweiligen der Aussparungen (141) aufgenommen wird und in Eingriff kommt, während es von einer der zwei benachbarten Sperren (15) an axialer Bewegung relativ zu dem zweiten Minderungsabschnitt (252) gehindert wird, wodurch der Nadelsitz (2) fest in die Gebrauchsposition gesetzt wird.

18. Einwegspritze nach Anspruch 17, **dadurch gekennzeichnet, dass** die Halteregion (14) eine Mehrzahl von Erhebungen (143) hat, die sich jeweils einwärts und radial von den Aussparungen (141) erstrecken, so dass, wenn die Trennwände (272) um den vorbestimmten Winkel in einer entsprechenden Richtung entgegen dem Uhrzeigersinn bzw. im Uhrzeigersinn gedreht werden, die Sperrsegmente (29) jeweils über die Erhebungen (143) gleiten, so dass der Benutzer sich der Bewegung der Sperrsegmente (29) auf eine Position bewusst ist, in der die Sperrsegmente (29) von den Sperren (15) nicht festgehalten werden und eine axiale Bewegung durchführen können.

19. Einwegspritze nach Anspruch 1, **dadurch gekennzeichnet, dass** das verformbare Dichtungselement (45) angeordnet ist, um von der vorderen Endwand (41) beabstandet zu sein, um relative Bewegung des verformbaren Dichtungselements (45) in Richtung auf die vordere Endwand (41) zuzulassen, wenn die Schubkraft zum Überwinden der zweiten Reibungskraft ausgeübt wird.

## Revendications

1. Seringue à usage unique comprenant:
un cylindre (1) possédant une paroi de cylindre environnante (12) qui entoure un axe (X) dans une direction longitudinale et qui définit un passage (11) à l'intérieur qui présente des extrémités ouvertes avant et arrière (123, 124) opposées l'une à l'autre, ladite paroi de cylindre environnante (12) ayant une surface de paroi interne (121) qui inclut des segments de surface avant et arrière (126, 125) à proximité desdites extrémités ouvertes avant et arrière (123, 124), respectivement, et un segment de surface intermédiaire (127) disposé entre ceux-ci;
une canule à aiguille (31);
un siège d'aiguille tubulaire (2) configuré pour être insérable dans ledit passage (11) à partir de ladite extrémité ouverte arrière (124) et entourant l'axe (X); et
un piston (4) disposé pour être mobile dans ledit passage (11) le long dudit segment de surface arrière (125), dans laquelle
ledit segment de surface intermédiaire (127) dudit cylindre (1) possède une région de retenue (14) qui est axialement disposée vers l'arrière à partir dudit segment de surface avant (126) et une première et une deuxième régions de frottement coniques (1271, 1272) qui sont respectivement interposées entre ladite région de retenue (14) et ledit segment de surface avant (126) et entre ladite région de retenue (14) et ledit segment de surface arrière (125);
ledit siège d'aiguille (2) incluant:
une partie d'engagement avant (22) qui est configurée pour être en engagement étanche aux fluides avec ledit segment de surface avant (126) et qui est disposée pour fixer ladite canule à aiguille (31) le long de l'axe (X),
une portion de retenue (23) qui est disposée vers l'arrière de ladite partie d'engagement avant (22) et qui est retenue au niveau de ladite région de retenue (14) en vertu d'une première force de frottement lorsque ledit siège d'aiguille (2) est dans une position d'utilisation,
une partie d'engagement arrière (25) qui s'étend vers l'arrière à partir de ladite région de retenue (23) pour terminer au niveau d'une paroi faisant face à l'arrière (212),
une collerette d'étanchéité environnante (251) qui s'étend à partir de ladite paroi faisant face à l'arrière (212) radialement et vers l'extérieur pour être en engagement étanche aux fluides avec ledit segment de surface arrière (125) et qui s'étend vers l'avant pour terminer au niveau d'une surface de collerette environnante (253) qui est mobile vers ladite deuxième région de frottement conique (1272) dans la direction longitudinale, ladite partie d'engagement arrière (25) ayant une surface de paroi tubulaire interne (261) qui entoure l'axe (X) pour définir une cavité (26) qui s'étend à partir de ladite paroi faisant face à l'arrière (212) vers ladite partie d'engagement avant (22) et qui termine au niveau d'une paroi supérieure (24), ladite paroi supérieure (24) possédant un trou axial (291) qui établit une communication de fluide entre ladite canule à aiguille (31) et ladite cavité (26), et
une languette déformable radialement (263) qui est disposé sur ladite surface de paroi tubulaire interne (261) vers l'arrière à partir de ladite paroi supérieure (24) et qui est déformable radialement et vers l'extérieur en réponse à une force de frottement cinétique;
ledit piston (4) possédant une paroi d'extrémité avant (41), une tête d'engagement (43) qui est opposée à ladite paroi d'extrémité avant (41) dans la direction longitudinale, un col (44) qui est interposé entre ladite tête d'engagement (43) et ladite paroi d'extrémité avant (41) et qui est d'une dimension telle qu'une paroi d'épaulement faisant face à l'arrière (431) est formée entre ladite tête d'engagement (43) et ledit col (44) et de sorte que la force de frottement cinétique est générée en conséquence d'un mouvement axial de ladite tête d'engagement (43) relativement à ladite languette déformable radialement (263) vers ladite paroi supérieure (24), et un élément étanche déformable (45) qui est manchonné sur ladite tête d'engagement (43) et ledit col (44), qui est en engagement par frottement avec ladite tête d'engagement (43) avec une deuxième force de frottement, et qui peut glisser de manière étanche relativement audit segment de surface arrière (125) de sorte que:
dans la position d'utilisation, ledit élément étanche déformable (45) est bougé vers l'avant en vertu d'un mouvement avant dudit piston (4) pour buter contre ladite paroi faisant face à l'arrière (212), tandis que ladite tête d'engagement (43) est portée plus loin dans ladite cavité (26), et de sorte que:
lorsque ledit piston (4) doit être placé dans une position d'élimination, ladite tête d'engagement (43) continue à bouger vers ladite paroi supérieure (24) par une force de poussée qui est appliquée sur ledit piston (4) et qui, lorsque ledit élément étanche déformable (45) est empêché par ladite paroi faisant face à l'arrière (212) de bouger avec ladite tête d'engagement (43), surpasse la deuxième force de frottement, exposant ainsi ledit col (44) de manière à permettre à ladite paroi d'épaulement faisant face à l'arrière (431) d'être forcée à glisser sur ladite languette déformable radialement et à être retenue par ladite languette déformable radialement (263) de sorte que ladite paroi d'épaulement faisant face à l'arrière (431) est empêchée de bouger vers l'arrière,
ladite tête d'engagement (43) et ladite surface de paroi tubulaire interne (261) étant configurée de sorte que, après le glissement de ladite paroi d'épaulement faisant face à l'arrière (431) sur ladite languette déformable radialement (263), un mouvement poursuivi de ladite tête d'engagement (43) vers ladite paroi supérieure (24), contre la première force de frottement, force ladite portion de retenue (23) et ladite collerette d'étanchéité environnante (251) à bouger au-delà desdites première et deuxième régions de frottement coniques (1271, 1272), respectivement, de manière à faciliter une action de traction subséquente dudit piston (4), en conséquence de quoi ledit siège d'aiguille (2) est amené vers ladite extrémité ouverte arrière (124) en vertu de la retenue de ladite paroi d'épaulement faisant face à l'arrière (431) par ladite languette déformable radialement (263), rétractant de cette manière ladite canule à aiguille (31) dans ledit passage (11).

2. Seringue jetable suivant la revendication 1, **caractérisée en ce que** ledit siège d'aiguille (2) est disposé pour être en engagement cannelé avec ladite tête d'engagement (43) de sorte que, une fois que ladite paroi d'épaulement faisant face à l'arrière (431) est retenue par ladite languette déformable radialement (263), et une fois que ladite portion de retenue (23) et ladite collerette d'étanchéité environnante (251) sont bougées au-delà desdites première et deuxième régions de frottement coniques (1271, 1272), respectivement, ledit siège d'aiguille (2) est autorisé à tourner avec ledit piston (4) pour ainsi diminuer encore le frottement entre ledit siège d'aiguille (2) et ladite surface de paroi interne (121) de manière à faciliter l'action de traction subséquente dudit piston (4).

3. Seringue jetable suivant la revendication 2, **caractérisée en ce que** ledit siège d'aiguille (2) possède une pluralité de nervures s'étendant axialement (262) qui sont formées sur ladite surface de paroi tubulaire interne (261) à proximité de ladite paroi supérieure (24) et qui sont déplacées angulairement les unes par rapport aux autres autour de l'axe (X), ladite tête d'engagement (43) possédant une pluralité de rainures s'étendant axialement (432) formées pour s'apparier avec lesdites nervures s'étendant axialement (262) de manière à amener ledit siège d'aiguille (2) en engagement cannelée avec ladite tête d'engagement (43).

4. Seringue jetable suivant la revendication 1, **caractérisée en ce que** ladite partie d'engagement avant (22) possède un trou de remplissage (292) en communication de fluide avec ledit trou axial (291) et rempli avec un adhésif pour fixer ladite canule à aiguille à ladite partie d'engagement avant (22).

5. Seringue jetable suivant la revendication 1, **caractérisée en ce que** ledit segment de surface intermédiaire possède une butée d'épaulement (13) qui est disposée entre ladite région de retenue (14) et ladite deuxième région de frottement conique (1272) pour permettre une butée de ladite surface de collerette environnante (253) contre celle-ci immédiatement après le déplacement de ladite collerette d'étanchéité environnante (251) au-delà de ladite deuxième région de frottement conique (1272).

6. Seringue jetable suivant la revendication 1, **caractérisée en ce que** ladite région de retenue (14) s'étendant radialement et vers l'extérieur pour former un creux annulaire (141), ledit siège d'aiguille (2) ayant une protubérance (28) formée sur ladite région de retenue (23) de manière à être retenu dans ledit creux (141) en vertu de la première force de frottement.

7. Seringue jetable suivant la revendication 6, **caractérisée en ce que** ladite portion de retenue (23) possède une pluralité d'ailettes (272) qui sont déplacées angulairement les unes par rapport aux autres autour de l'axe (X) et qui sont espacées de ladite première région de frottement conique radialement, chacune desdites ailettes (272) étant configurée de sorte qu'un contour constitué par lesdites ailettes (272) autour de l'axe (X) fait office de ladite protubérance (28).

8. Seringue jetable suivant la revendication 6, **caractérisée en ce que** ledit segment de surface intermédiaire (127) possède une pluralité d'ailettes (131) qui sont déplacées angulairement les unes par rapport aux autres autour de l'axe (X) et qui s'étendent vers l'arrière pour être espacées de ladite collerette d'étanchéité environnante (251) par ladite deuxième région de frottement conique (1272), chacune desdites ailettes (131) s'étendant radialement et vers l'intérieur pour terminer à une extrémité distale (1311), lesdites extrémités distales (1311) étant configurées de sorte qu'un contour constitué par lesdites extrémités distales (1311) autour de l'axe (X) fait office dudit creux (141).

9. Seringue jetable suivant la revendication 1, **caractérisée en ce que** ledit segment de surface avant (126) est configuré pour converger vers ladite extrémité ouverte avant (123) pour empêcher le retrait dudit siège d'aiguille (2) à partir de ladite extrémité ouverte avant (123).

10. Seringue jetable suivant la revendication 1, **caractérisée en ce que** ladite paroi de cylindre environnante (12) possède une surface de paroi externe (122) qui a une portion de nervures (129) s'étendant dans la direction longitudinale et disposée adjacente à ladite extrémité ouverte avant (123), ladite seringue jetable comprenant en outre un protecteur de pointe (32) qui est disposé pour être manchonné sur ladite surface de paroi externe (122) et qui est retenu par frottement par ladite portion de nervures (129) pour protéger ladite canule à aiguille (31).

11. Seringue jetable suivant la revendication 1, **caractérisée en outre par** un joint torique d'étanchéité (5) qui est disposé sur ledit siège d'aiguille (2), qui entoure l'axe (X) et qui est configuré pour augmenter l'étanchéité aux fluides de l'engagement entre ledit segment de surface avant (126) et ladite partie d'engagement avant (22) ou entre ledit segment de surface arrière (125) et ladite collerette d'étanchéité environnante (251).

12. Seringue jetable suivant la revendication 1, **caractérisée en ce que** ladite paroi de cylindre environnante (12) possède une surface de paroi externe (122) qui a une portion de palier annulaire (120) qui fait face à l'avant et qui est distale à ladite extrémité ouverte avant (123) et un segment avant environnant (1221) interposé entre ladite extrémité ouverte avant (123) et ladite portion de palier annulaire (120), ladite seringue jetable comprenant en outre:
un embout de cathéter (33) incluant une paroi d'embout environnante qui possède une portion de manchon qui est manchonnée sur ledit segment avant environnant (1221) et qui a un bord terminal butant contre ladite portion de palier (120) et une portion de pointe opposée à ladite portion de manchon le long de l'axe (X); et
un cathéter tubulaire (34) incluant un segment proximal qui est disposé dans ladite portion de pointe et qui s'étend le long de l'axe (X) et un segment distal qui s'étend à partir dudit segment proximal le long de l'axe (X) pour se projeter vers l'extérieur de ladite portion de pointe.

13. Seringue jetable suivant la revendication 1, **caractérisée en ce que** ladite partie d'engagement avant (22) possède un segment de siège qui est configuré pour s'étendre vers l'extérieur dudit cylindre (1) à partir de ladite extrémité ouverte avant (123) lorsque ledit siège d'aiguille (2) est dans la position d'utilisation.

14. Seringue jetable suivant la revendication 13, **caractérisée en outre par** un protecteur de pointe (32) qui est disposé pour être manchonné sur et qui est retenu par frottement audit segment de siège de ladite partie d'engagement avant (22) pour protéger ladite canule à aiguille (31).

15. Seringue jetable suivant la revendication 13, **caractérisée en ce que** ladite partie d'engagement avant (22) possède en outre un segment d'embout (35) qui est configuré pour fixer ladite canule à aiguille le long de l'axe (X) et qui est manchonné sur ledit segment de siège pour établir une communication de fluide entre ladite canule à aiguille (31 ) et ledit trou axial (291).

16. Seringue jetable suivant la revendication 15, **caractérisée en outre par**:
un embout de cathéter (33) incluant une paroi d'embout environnante qui possède une portion de manchon qui est manchonnée sur ledit segment d'embout (35) et une portion de pointe opposée à ladite portion de manchon le long de l'axe (X); et
un cathéter tubulaire (34) incluant un segment proximal qui est disposé dans ladite portion de pointe et qui s'étend le long de l'axe (X) et un segment distal qui s'étend à partir dudit segment proximal le long de l'axe (X) et vers l'extérieur de ladite portion de pointe.

17. Seringue jetable suivant la revendication 13, **caractérisée en ce que** ladite région de retenue (14) possède une pluralité de creux (141) qui sont déplacés angulairement les uns par rapport aux autres autour de l'axe (X),
ledit segment de surface intermédiaire (127) ayant en outre une pluralité de barrières (15) qui sont déplacées angulairement les unes par rapport aux autres autour de l'axe (X),
ladite portion de retenue (23) dudit siège d'aiguille (2) possédant une pluralité de séparations (272) qui sont déplacées angulairement les unes par rapport aux autres autour de l'axe (X) et une pluralité de segments bloquants (29) qui s'étendent respectivement à partir desdites séparations (272) radialement et vers l'extérieur, chacun desdits segments bloquants (29) étant configuré de sorte que, lorsque ledit siège d'aiguille (2) est amené pour être inséré dans ledit passage (11) à partir de ladite extrémité ouverte arrière (124) et immédiatement après que chacune desdites séparations (272) est amenée à passer entre deux adjacentes desdites barrières (15), chacune desdites séparations (272) est tournée à un angle prédéterminé dans une des directions dans le sens des aiguilles d'une montre ou inverse des aiguilles d'une montre de sorte que chacun desdits segments bloquants (29) est reçu dans et en engagement avec un respectif desdits creux (141) en vertu de la première force de frottement, tout en étant empêché par une desdites deux adjacentes desdites barrières (15) d'un mouvement axial relativement à ladite deuxième région conique (252), plaçant ainsi ledit siège d'aiguille (2) fermement dans la position d'utilisation.

18. Seringue jetable suivant la revendication 17, **caractérisée en ce que** ladite région de retenue (14) possède une pluralité de bosses (143) s'étendant respectivement à partir desdits creux (141) vers l'intérieur et radialement de sorte que lesdites séparations (272) sont tournées à l'angle prédéterminé dans une des directions dans le sens des aiguilles d'une montre et inverse des aiguilles d'une montre correspondante, lesdits segments bloquants (29) glissent sur lesdites bosses (143) respectivement de sorte que l'utilisateur a conscience du mouvement desdits segments bloquants (29) vers une position où lesdits segments bloquants (29) ne sont pas restreints par lesdites barrières (15) et sont autorisés à effectuer un mouvement axial.

19. Seringue jetable suivant la revendication 1, **caractérisée en ce que** ledit élément étanche déformable (45) est disposé pour être espacé de ladite paroi d'extrémité avant (41) de manière à permettre un mouvement relatif dudit élément étanche déformable (45) vers ladite paroi d'extrémité avant (41) lorsque la force de poussée est appliquée pour surpasser la deuxième force de frottement.
